(19) Europäisches Patentamt
European Patent Office
Office européen des brevets

(11) **EP 4 140 533 B1**

(12) **EUROPEAN PATENT SPECIFICATION**

(45) Date of publication and mention
of the grant of the patent:
**08.04.2026 Bulletin 2026/15**

(21) Application number: **22190931.0**

(22) Date of filing: **18.08.2022**

(51) International Patent Classification (IPC):
**A61N 1/36** (2006.01)

(52) Cooperative Patent Classification (CPC):
**A61N 1/3614; A61N 1/36142; A61N 1/0534**

(54) **TEMPLATE-BASED DETERMINATION OF ELECTROPHYSIOLOGICAL SIGNAL SOURCES**

VORLAGENBASIERTE BESTIMMUNG VON ELEKTROPHYSIOLOGISCHEN SIGNALQUELLEN

DÉTERMINATION DE SOURCES DE SIGNAL ÉLECTROPHYSIOLOGIQUE BASÉE SUR DES MODÈLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.08.2021 US 202117410268**

(43) Date of publication of application:
**01.03.2023 Bulletin 2023/09**

(73) Proprietor: **Medtronic, Inc.
Minneapolis, Minnesota 55432 (US)**

(72) Inventors:
• **Jackson, Jadin C.
Minneapolis, 55432 (US)**
• **Panken, Eric J.
Minneapolis, 55432 (US)**
• **Case, Michelle A.
Minneapolis, 55432 (US)**
• **Molina, Rene A.
Minneapolis, 55432 (US)**
• **Becker, Abbey Beuning Holt
Minneapolis, 55432 (US)**
• **Pulliam, Christopher L.
Minneapolis, 55432 (US)**

(74) Representative: **Zimmermann & Partner
Patentanwälte mbB
Postfach 330 920
80069 München (DE)**

(56) References cited:
**WO-A1-2007/097872        US-A1- 2011 264 165
US-A1- 2017 259 064        US-A1- 2018 071 530**

**Description**

TECHNICAL FIELD

This disclosure generally relates to electrical stimulation and recording.

BACKGROUND

Medical devices may be external or implanted, and may be used to deliver electrical stimulation therapy to various tissue sites of a patient to treat a variety of symptoms or conditions such as chronic pain, tremor, Parkinson's disease, other movement disorders, epilepsy, urinary or fecal incontinence, sexual dysfunction, obesity, or gastroparesis. A medical device may deliver electrical stimulation therapy via one or more leads that include electrodes located proximate to target locations associated with the brain, the spinal cord, pelvic nerves, peripheral nerves, or the gastrointestinal tract of a patient. Hence, electrical stimulation may be used in different therapeutic applications, such as deep brain stimulation (DBS), spinal cord stimulation (SCS), pelvic stimulation, gastric stimulation, or peripheral nerve field stimulation (PNFS).

A clinician may select values for a number of programmable parameters in order to define the electrical stimulation therapy to be delivered by the implantable stimulator to a patient. For example, the clinician may select one or more electrodes for delivery of the stimulation, a polarity of each selected electrode, a voltage or current amplitude, a pulse width, and a pulse frequency as stimulation parameters. A set of parameters, such as a set including electrode combination, electrode polarity, voltage or current amplitude, pulse width and pulse rate, may be referred to as a program in the sense that they define the electrical stimulation therapy to be delivered to the patient. Document US 2018/071530 A1 relates to electrical stimulation devices.

SUMMARY

The invention is defined by the appended claims. In general, the disclosure describes devices, systems, and techniques related to managing electrical stimulation and/or sensing of physiological signals. For example, an implantable medical device (IMD) may be coupled to one or more leads carrying an array of electrodes. The IMD may monitor electrical signals sensed by different electrode combinations to determine an estimated location of a signal source of interest and/or determine values of one or more stimulation parameters for subsequent electrical stimulation. Patient conditions or symptoms may be caused or associated with various neurological activity, such as activity at certain locations within a brain of the patient. In some examples, electrical stimulation may be delivered to affect these locations to reduce or eliminate one or more patient symptoms or conditions.

The system may sense electrical signals from multiple different electrode combinations of one or more leads. The system may extract features from these electrical signals and determine an estimated location of a signal source (e.g., a target anatomical structure from which physiological signals originate or propagate) to receive electrical stimulation. The system may then process these features from the sensed electrical signals in order to determine an estimated location of a signal source with respect to one or more leads. For example, the system may compare the features to one or more templates that describe relationships between features of sensed electrical signals and locations of signal sources with respect to one or more leads. Based on this comparison, the system may select the estimated location and display the estimated location via a user interface and/or determine one or more stimulation parameter values that define electrical stimulation to target the estimated location of the signal source.

In one example, a system includes processing circuitry configured to receive information representing a plurality of signals sensed from a tissue of a patient via a plurality of electrode combinations, wherein the plurality of electrode combinations comprises different electrode combinations comprising electrodes disposed at different positions of the lead implanted in the patient; determine one or more features from the information representing the plurality of signals; compare the one or more features to a plurality of templates, each template of the plurality of templates representing respective locations of a signal source within the tissue; and determine, based on the comparison of the one or more features to the plurality of templates, an estimated location of the signal source with respect to the lead.

In another example, a method includes receiving, by processing circuitry, information representing a plurality of signals sensed from a tissue of a patient via a plurality of electrode combinations, wherein the plurality of electrode combinations comprises different electrode combinations comprising electrodes disposed at different positions of the lead implanted in the patient; determining, by the processing, one or more features from the information representing the plurality of signals; comparing, by the processing circuitry, the one or more features to a plurality of templates, each template of the plurality of templates representing respective locations of a signal source within the tissue; and determining, by the processing circuitry, based on the comparison of the one or more features to the plurality of templates, an estimated location of the signal source with respect to the lead.

In another example, a computer-readable storage medium comprising instructions that, when executed, cause processing circuitry to receive information representing a plurality of signals sensed from a tissue of a patient via a plurality of electrode combinations, wherein the plurality of electrode combinations comprises different electrode combinations comprising electrodes disposed at different positions of the lead implanted in the patient; determine one or more features from the information representing the plurality of signals; compare the one or more features to a plurality of templates, each template of the plurality of templates representing respective locations of a signal source within the tissue; and determine, based on the comparison of the one or more features to the plurality of templates, an estimated location of the signal source with respect to the lead.

The details of one or more examples of the techniques of this disclosure are set forth in the accompanying drawings and the description below. Other features, objects, and advantages of the techniques will be apparent from the description and drawings, and from the claims.

BRIEF DESCRIPTION OF DRAWINGS

FIG. 1 is a conceptual diagram illustrating an example system that includes an implantable medical device (IMD) configured to deliver DBS to a patient according to an example of the techniques of the disclosure.

FIG. 2 is a block diagram of the example IMD of FIG. 1 for delivering DBS therapy according to an example of the techniques of the disclosure.

FIG. 3 is a block diagram of the external programmer of FIG. 1 for controlling delivery of DBS therapy according to an example of the techniques of the disclosure.

FIGS. 4A and 4B are conceptual diagrams of example leads with respective electrodes carried by the lead.

FIGS. 5A, 5B, 5C, and 5D are conceptual diagrams of example electrodes disposed around a perimeter of a lead at a particular longitudinal location.

FIG. 6 is a coronal view of example tissue with a lead placed with respect to a target location within tissue.

FIG. 7 is an axial view of example tissue with a lead placed with respect to a target location within tissue.

FIGS. 8A, 8B, 8C, and 8D are conceptual views of an example lead with example initial information and signal information recorded from respective electrode combinations.

FIG. 9A is a conceptual illustration of an example signal source estimated location with respect to electrodes of a lead.

FIG. 9B is a conceptual illustrations of an example electrical field that can be generated based on an estimated location of the signal source.

FIGS. 10A and 10B are example graphs illustrating example sensed voltages that vary with distance from electrode combinations.

FIGS. 11A and 11B are example graphs illustrating estimated locations for a signal source compared with actual locations of the signal source.

FIG. 12 is a flowchart illustrating an example technique for generating templates of one or more features from sensed signals for different electrode combinations and signal source locations with respect to a lead.

FIG. 13 is a flowchart illustrating an example technique for determining values of one or more stimulation parameters that define electrical stimulation deliverable based on the estimated location of a signal source with respect to a lead.

DETAILED DESCRIPTION

This disclosure describes various devices, systems, and techniques for determining estimated locations of signals sources in tissue and, in some examples, selecting stimulation parameters that define electrical stimulation to affect neural tissue at the estimated location of the signal source. A patient may suffer from one or more symptoms treatable by electrical stimulation therapy. For example, a patient may suffer from brain disorder such as Parkinson's disease, Alzheimer's disease, or another type of movement disorder. Deep brain stimulation (DBS) may be an effective treatment to reduce the symptoms associated with such disorders.

However, efficacy of stimulation therapy may be reliant on selecting appropriate electrodes and other stimulation parameter values that direct an electric field to a target region of tissue. Stimulation of tissue outside of the target region and/or with parameter values too low or too high may elicit undesirable effects and/or reduce the efficacy of the therapy. In addition, a lead, and the electrodes it carries, may move within tissue after implantation. Therefore, the system may benefit from identifying where the target region of tissue resides with respect to the lead (e.g., where the electrodes are positioned with respect to the target region of tissue). In addition, if a lead rotates about a longitudinal axis and/or shifts longitudinally within tissue after stimulation parameters are determined, the stimulation therapy may be less effective and/or the stimulation may result in undesirable side effects for the patient. Further, if the patient's disease progresses or otherwise has physiological changes that occur over time, the prior stimulation parameters may no longer provide effective therapy to treat symptoms or cause new side effects.

As described herein, various devices, systems, and techniques may determine estimated locations of a signal source in tissue with respect to one or more leads, display the estimated location via a user interface, and/or determine values of one or more stimulation parameters (e.g., electrode combinations and/or current amplitudes) to use for delivering stimulation therapy. A lead may carry a plurality of electrodes at different longitudinal positions and, in some examples, at different positions around the longitudinal axis and the perimeter of the lead. An implantable medical device (e.g., an IMD) may be configured to monitor electrical signals (e.g., LFPs) sensed by different electrode combinations. Certain networks or collection of neurons within tissue may contribute to patient conditions and symptoms. These target tissues are thus located at different distances and orientations with respect to electrodes of one or more leads. In order to provide therapy to a patient, the clinician or system may benefit from identifying where these target tissues are located with respect to one or more leads in order to select appropriate stimulation parameter values, such as electrode combination, current amplitudes, pulse width, pulse frequency, etc.

For example, the system (e.g., the IMD, an external programmer, another device, or some combination thereof) may monitor electrical signals sensed by different electrode combinations to determine an estimated location of a signal source (e.g., a location of neurons that produce the signal source) of interest and/or determine values of one or more stimulation parameters for subsequent electrical stimulation. Patient conditions or symptoms may be caused or associated with various neurological activity, such as activity at certain locations within a brain of the patient. The system may sense electrical signals from multiple different electrode combinations of one or more leads. The system may extract features (e.g., voltage amplitudes, spectral power, one or more frequency bands, etc.) from these electrical signals and determine an estimated location of a signal source (e.g., a target anatomical structure from which physiological signals originate or propagate) to receive electrical stimulation. The system may then process these features from the sensed electrical signals in order to determine an estimated location of a signal source with respect to one or more leads. For example, the system may compare the features to one or more templates that describe relationships between features of sensed electrical signals and locations of signal sources with respect to one or more leads. The system may perform this comparison by way of comparing the features to a look up table stored in memory and/or solving a cost function that derives the estimated location based on the features from the electrode combinations. Based on this comparison, the system may select the estimated location of the signal source that represents the target tissue for stimulation, for example. The system may control a user interface to display the estimated location to the patient. In some examples, the system may determine, based on the estimated location with respect to one or more leads, one or more stimulation parameter values that define electrical stimulation to target the estimated location of the signal source. In addition, the system may compare this estimated location to previously identified estimated locations to determine that the lead has migrated within the tissue, such as moved axially or rotated circumferentially over time.

For example, the system may determine initial information representing electrical signals sensed by different electrode combinations at a first time, such as just after implantation or programming. The system may then determine the estimated location of the signal source for use display and/or determining stimulation parameter values. The system may periodically (e.g., at regular intervals or in response to a trigger event indicative of a possible lead movement) determine other signal information representing electrical signals sensed by the different electrode combinations at a second time after the first time. The system may then determine the new estimated location with respect to one or more leads, which may be the same or different than the previous estimated location, and adjust stimulation parameter values if needed. In some examples, the system can determine that the electrodes have moved with respect to tissue or that the disease has improving or worsening (e.g., based on signal source location movement and/or signal changes to frequency or amplitude).

Although this disclosure is directed to DBS therapy, the systems, devices, and techniques described herein may similarly determine signal source locations with respect to leads and electrodes implanted outside of the brain, such as near other nerves or muscles for different diagnostic or therapeutic applications, such as spinal cord stimulation (SCS), pelvic stimulation, gastric stimulation, or peripheral nerve field stimulation (PNFS). Moreover, a human patient is described for example purposes herein, but similar systems, devices, and techniques may be used for other animals in other examples.

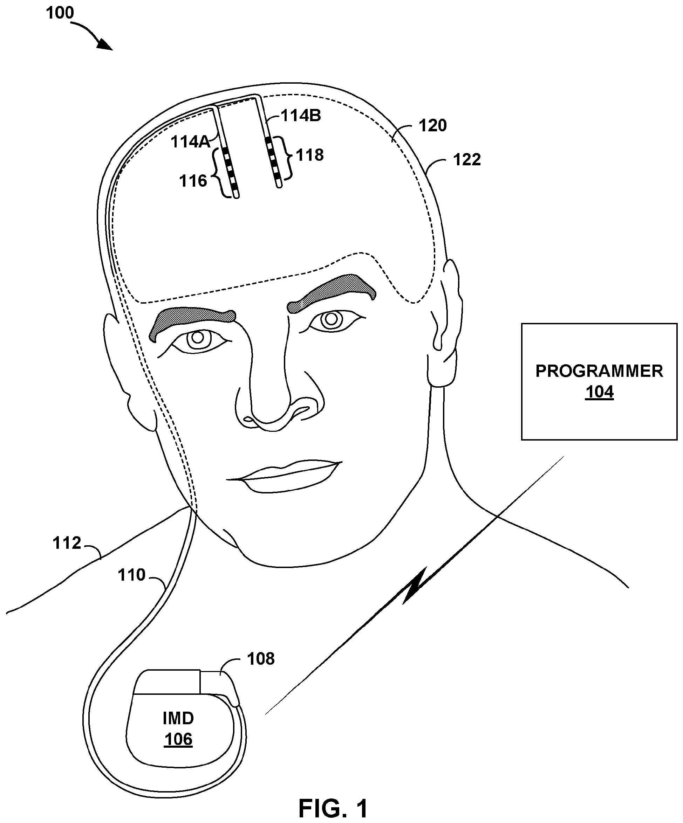

FIG. 1 is a conceptual diagram illustrating an example system 100 that includes an implantable medical device (IMD) 106 configured to deliver DBS to patient 122 according to an example of the techniques of the disclosure. As shown in the example of FIG. 1, example system 100 includes medical device programmer 104, implantable medical device (IMD) 106, lead extension 110, and leads 114A and 114B with respective sets of electrodes 116, 118. In the example shown in FIG. 1, electrodes 116, 118 of leads 114A, 114B are positioned to deliver electrical stimulation to a tissue site within brain 120, such as a deep brain site under the dura mater of brain 120 of patient 112. In some examples, delivery of stimulation to one or more regions of brain 120, such as the subthalamic nucleus, globus pallidus or thalamus, may be an effective treatment to manage movement disorders, such as Parkinson's disease. Some or all of electrodes 116, 118 also may be positioned to sense neurological brain signals within brain 120 of patient 112. In some examples, some of electrodes 116, 118 may be configured to sense neurological brain signals and others of electrodes 116, 118 may be configured to deliver adaptive electrical stimulation to brain 120. In other examples, all of electrodes 116, 118 are configured to both sense neurological brain signals and deliver adaptive electrical stimulation to brain 120.

IMD 106 includes a therapy module (e.g., which may include processing circuitry, signal generation circuitry or other electrical circuitry configured to perform the functions attributed to IMD 106) that includes a stimulation generator configured to generate and deliver electrical stimulation therapy to patient 112 via a subset of electrodes 116, 118 of leads 114A and 114B, respectively. The subset of electrodes 116, 118 that are used to deliver electrical stimulation to patient 112, and, in some cases, the polarity of the subset of electrodes 116, 118, may be referred to as a stimulation electrode combination. As described in further detail below, the stimulation electrode combination can be selected for a particular patient 112 and target tissue site (e.g., selected based on the patient condition or based on the detected location of a signal source). The group of electrodes 116, 118 includes at least one electrode and can include a plurality of electrodes. In some examples, the plurality of electrodes 116 and/or 118 may have a complex electrode geometry such that two or more electrodes of the lead are located at different positions around the perimeter of the respective lead (e.g., different positions around a longitudinal axis of the lead).

In some examples, the neurological signals (e.g., an example type of electrical signals) sensed within brain 120 may reflect changes in electrical current produced by the sum of electrical potential differences across brain tissue. Examples of neurological brain signals include, but are not limited to, electrical signals generated from local field potentials (LFP) sensed within one or more regions of brain 120, electroencephalogram (EEG) signals, or electrocorticogram (ECoG) signals. Any of these sensed signals may be intrinsic signals generated by physiological neural activity and/or evoked signals generated in response to a delivered stimulus (e.g., a delivered electrical stimulation signal).

In some examples, the neurological brain signals that are used to select a stimulation electrode combination may be sensed within the same region of brain 120 as the target tissue site for the electrical stimulation. As previously indicated, these tissue sites may include tissue sites within anatomical structures such as the thalamus, subthalamic nucleus or globus pallidus of brain 120, as well as other target tissue sites. The specific target tissue sites and/or regions within brain 120 may be selected based on the patient condition. Thus, due to these differences in target locations, in some examples, the electrodes used for delivering electrical stimulation may be different than the electrodes used for sensing neurological brain signals. In other examples, the same electrodes may be used to deliver electrical stimulation and sense brain signals. However, this configuration would require the system to switch between stimulation generation and sensing circuitry and may reduce the time the system can sense brain signals.

Electrical stimulation generated by IMD 106 may be configured to manage a variety of disorders and conditions. In some examples, the stimulation generator of IMD 106 is configured to generate and deliver electrical stimulation pulses to patient 112 via electrodes of a selected stimulation electrode combination. However, in other examples, the stimulation generator of IMD 106 may be configured to generate and deliver a continuous wave signal, e.g., a sine wave or triangle wave. In either case, a stimulation generator within IMD 106 may generate the electrical stimulation therapy for DBS according to a therapy program that is selected at that given time in therapy. In examples in which IMD 106 delivers electrical stimulation in the form of stimulation pulses, a therapy program may include a set of therapy parameter values (e.g., stimulation parameters), such as a stimulation electrode combination for delivering stimulation to patient 112, pulse frequency, pulse width, and a current or voltage amplitude of the pulses. As previously indicated, the electrode combination may indicate the specific electrodes 116, 118 that are selected to deliver stimulation signals to tissue of patient 112 and the respective polarities of the selected electrodes. IMD 106 may deliver electrical stimulation intended to contribute to a therapeutic effect. In some examples, IMD 106 may also, or alternatively, deliver electrical stimulation intended to be sensed by other electrode and/or elicit a physiological response, such as an evoked compound action potential (ECAP), that can be sensed by electrodes.

IMD 106 may be implanted within a subcutaneous pocket above the clavicle, or, alternatively, on or within cranium 122 or at any other suitable site within patient 112. Generally, IMD 106 is constructed of a biocompatible material that resists corrosion and degradation from bodily fluids. IMD 106 may comprise a hermetic housing to substantially enclose components, such as a processor, therapy module, and memory.

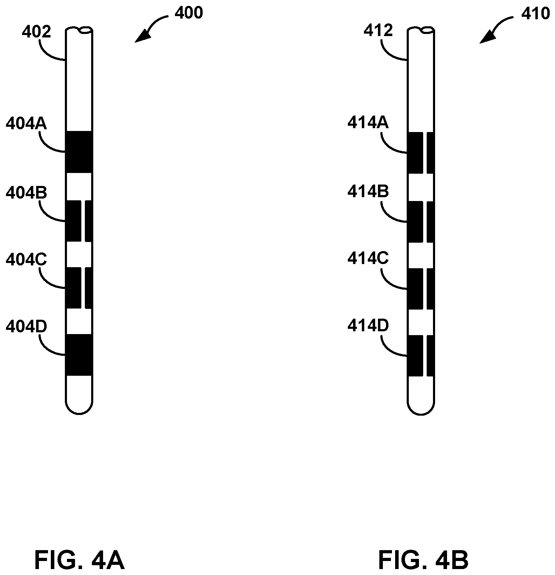

As shown in FIG. 1, implanted lead extension 110 is coupled to IMD 106 via connector 108 (also referred to as a connector block or a header of IMD 106). In the example of FIG. 1, lead extension 110 traverses from the implant site of IMD 106 and along the neck of patient 112 to cranium 122 of patient 112 to access brain 120. In the example shown in FIG. 1, leads 114A and 114B (collectively "leads 114") are implanted within the right and left hemispheres, respectively, of patient 112 in order deliver electrical stimulation to one or more regions of brain 120, which may be selected based on the patient condition or disorder controlled by therapy system 100. The specific target tissue site and the stimulation electrodes used to deliver stimulation to the target tissue site, however, may be selected, e.g., according to the identified patient behaviors and/or other sensed patient parameters. Other lead 114 and IMD 106 implant sites are contemplated. For example, IMD 106 may be implanted on or within cranium 122, in some examples. Or leads 114 may be implanted within the same hemisphere or IMD 106 may be coupled to a single lead implanted in a single hemisphere. Although leads 114 may have ring electrodes at different longitudinal positions as shown in FIG. 1, leads 114 may have electrodes disposed at different positions around the perimeter of the lead (e.g., different circumferential positions for a cylindrical shaped lead) as shown in the examples of FIGS. 4A and 4B.

Leads 114 illustrate an example lead set that include axial leads carrying ring electrodes disposed at different axial positions (or longitudinal positions). In other examples, leads may be referred to as "paddle" leads carrying planar arrays of electrodes on one side of the lead structure. In addition, as described herein, complex lead array geometries may be used in which electrodes are disposed at different respective longitudinal positions and different positions around the perimeter of the lead. As described herein, IMD 106 may be configured to detect movement of the lead with respect to tissue when monitoring electrical signals sensed by the different electrodes between different times.

Although leads 114 are shown in FIG. 1 as being coupled to a common lead extension 110, in other examples, leads 114 may be coupled to IMD 106 via separate lead extensions or directly to connector 108. Leads 114 may be positioned to deliver electrical stimulation to one or more target tissue sites within brain 120 to manage patient symptoms associated with a movement disorder of patient 112. Leads 114 may be implanted to position electrodes 116, 118 at desired locations of brain 120 through respective holes in cranium 122. Leads 114 may be placed at any location within brain 120 such that electrodes 116, 118 are capable of providing electrical stimulation to target tissue sites within brain 120 during treatment. For example, electrodes 116, 118 may be surgically implanted under the dura mater of brain 120 or within the cerebral cortex of brain 120 via a burr hole in cranium 122 of patient 112, and electrically coupled to IMD 106 via one or more leads 114.

In the example shown in FIG. 1, electrodes 116, 118 of leads 114 are shown as ring electrodes. Ring electrodes may be used in DBS applications because they are relatively simple to program and are capable of delivering an electrical field to any tissue adjacent to electrodes 116, 118. In other examples, electrodes 116, 118 may have different configurations. For example, in some examples, at least some of the electrodes 116, 118 of leads 114 may have a complex electrode array geometry that is capable of producing shaped electrical fields. The complex electrode array geometry may include multiple electrodes (e.g., partial ring or segmented electrodes) around the outer perimeter of each lead 114, rather than one ring electrode, such as shown in FIGS. 4A and 4B. In this manner, electrical stimulation may be directed in a specific direction from leads 114 to enhance therapy efficacy and reduce possible adverse side effects from stimulating a large volume of tissue. In some examples, a housing of IMD 106 may include one or more stimulation and/or sensing electrodes. In alternative examples, leads 114 may have shapes other than elongated cylinders as shown in FIG. 1. For example, leads 114 may be paddle leads, spherical leads, bendable leads, or any other type of shape effective in treating patient 112 and/or minimizing invasiveness of leads 114.

In the example shown in FIG. 1, IMD 106 includes a memory to store a plurality of therapy programs that each define a set of therapy parameter values. In some examples, IMD 106 may select a therapy program from the memory based on various parameters, such as sensed patient parameters and the identified patient behaviors. IMD 106 may generate electrical stimulation based on the selected therapy program to manage the patient symptoms associated with a movement disorder.

External programmer 104 wirelessly communicates with IMD 106 as needed to provide or retrieve therapy information. Programmer 104 is an external computing device that the user, e.g., a clinician and/or patient 112, may use to communicate with IMD 106. For example, programmer 104 may be a clinician programmer that the clinician uses to communicate with IMD 106 and program one or more therapy programs for IMD 106. Alternatively, programmer 104 may be a patient programmer that allows patient 112 to select programs and/or view and modify therapy parameters. The clinician programmer may include more programming features than the patient programmer. In other words, more complex or sensitive tasks may only be allowed by the clinician programmer to prevent an untrained patient from making undesirable changes to IMD 106. IMD 106 may also transmit notifications to programmer 104 for delivery to a user in response to detecting that one of leads 114 has moved with respect to tissue. Programmer 104 may enter a new programming session for the user to select new stimulation parameters for subsequent therapy. External programmer 104 may display estimated locations of target tissue locations and/or suggested stimulation parameter values for delivering electrical stimulation that affects the target tissue location.

When programmer 104 is configured for use by the clinician, programmer 104 may be used to transmit initial programming information to IMD 106. This initial information may include hardware information, such as the type of leads 114 and the electrode arrangement, the position of leads 114 within brain 120, the configuration of electrode array 116, 118, initial programs defining therapy parameter values, and any other information the clinician desires to program into IMD 106. Programmer 104 may also be capable of completing functional tests (e.g., measuring the impedance of electrodes 116, 118 of leads 114). In some examples, programmer 104 may receive sensed signals or representative information and perform the same techniques and functions attributed to IMD 106 herein. In other examples, a remote server (e.g., a standalone server or part of a cloud service) may perform the functions attributed to IMD 106, programmer 104, or any other devices described herein.

The clinician may also store therapy programs within IMD 106 with the aid of programmer 104. During a programming session, the clinician may determine one or more therapy programs that may provide efficacious therapy to patient 112 to address symptoms associated with the patient condition, and, in some cases, specific to one or more different patient states, such as a sleep state, movement state or rest state. For example, the clinician may select one or more stimulation electrode combination with which stimulation is delivered to brain 120. During the programming session, the clinician may evaluate the efficacy of the specific program being evaluated based on feedback provided by patient 112 or based on one or more physiological parameters of patient 112 (e.g., muscle activity, muscle tone, rigidity, tremor, etc.). Alternatively, identified patient behavior from video information may be used as feedback during the initial and subsequent programming sessions. Programmer 104 may assist the clinician in the creation/identification of therapy programs by providing a methodical system for identifying potentially beneficial therapy parameter values.

Programmer 104 may also be configured for use by patient 112. When configured as a patient programmer, programmer 104 may have limited functionality (compared to a clinician programmer) in order to prevent patient 112 from altering critical functions of IMD 106 or applications that may be detrimental to patient 112. In this manner, programmer 104 may only allow patient 112 to adjust values for certain therapy parameters or set an available range of values for a particular therapy parameter.

Programmer 104 may also provide an indication to patient 112 when therapy is being delivered, when patient input has triggered a change in therapy or when the power source within programmer 104 or IMD 106 needs to be replaced or recharged. For example, programmer 112 may include an alert LED, may flash a message to patient 112 via a programmer display, generate an audible sound or somatosensory cue to confirm patient input was received, e.g., to indicate a patient state or to manually modify a therapy parameter.

Therapy system 100 may be implemented to provide chronic stimulation therapy to patient 112 over the course of several months or years. However, system 100 may also be employed on a trial basis to evaluate therapy before committing to full implantation. If implemented temporarily, some components of system 100 may not be implanted within patient 112. For example, patient 112 may be fitted with an external medical device, such as a trial stimulator, rather than IMD 106. The external medical device may be coupled to percutaneous leads or to implanted leads via a percutaneous extension. If the trial stimulator indicates DBS system 100 provides effective treatment to patient 112, the clinician may implant a chronic stimulator within patient 112 for relatively long-term treatment.

Although IMD 106 is described as delivering electrical stimulation therapy to brain 120, IMD 106 may be configured to direct electrical stimulation to other anatomical regions of patient 112 in other examples. In other examples, system 100 may include an implantable drug pump in addition to, or in place of, IMD 106. Further, an IMD may provide other electrical stimulation such as spinal cord stimulation to treat a movement disorder.

According to the techniques of the disclosure, system 100 may initially upon implantation and/or periodically over time, sense electrical signals from a plurality of electrode combinations using electrodes on one or both of leads 114. System 100 may then determine one or more features of the sensed electrical signals and determine an estimated location of a signal source based on the one or more features. For example, system 100 may compare the one or more features of the sensed signals to a plurality of templates stored in memory. Each template may define a relationship between one or more features of sensed signals from one or more electrode combinations and the location of a signal source with respect to the one or more electrode combinations (e.g., the location of the signal source with respect to a lead that carries the electrodes of the electrode combinations). System 100 may generate or otherwise obtain these templates from modeled data, tank data (e.g., a lead placed in an experimental tank condition), or data from other patients (e.g., via prior data that may be subject to machine learning using this as training data or other analysis). System 100 may then display a representation of the estimated location of the signal source based on the sensed signals and templates and/or determine stimulation parameter values for subsequent stimulation therapy to modulate activity of the target tissue of the signal source.

System 100 (e.g., IMD 106) may also include processing circuitry configured to receive signal information indicative of electrical signals sensed from the plurality of electrode combinations at one or more times. The processing circuitry or sensing circuitry may generate the signal information based on the sensing circuitry sensing potential differences for each electrode combination. IMD 106 may then determine, based on the signal information and templates, new estimated locations of the signal source and/or adjustments to the stimulation parameter values. In some examples, system 100 may determine that a lead has rotated or moved axially with respect to tissue in response to determining that the estimated location has changed. To identify this situations, system 100 may sense electrical signals from the electrode combinations and redetermine the estimated location of a signal source in response to detecting a trigger event that could be indicative of lead movement, such as changes to stimulation therapy efficacy, changes to side effects, or any other sensed data or user feedback. System 100 may output an indication that the lead has moved with respect to the tissue in response to detecting that the estimated location has changed with respect to the lead.

In response to IMD 106 and/or programmer 104 determining that the lead has moved, IMD 106 may perform an action. For example, IMD 106 may control a display to present the indication to a user that the lead has rotated with respect to the tissue, which may or more not show the new estimated location and, in some examples, the old estimated location. Controlling the display may involve transmitting an alert to external programmer 104 which in turn presents the alert on the display of programmer 104. In some examples, IMD 106 may transmit a request to a user to update stimulation parameter values that define electrical stimulation because of the new estimated location of the signal source that may result in insufficiency therapy to the patient and/or cause undesirable side effects. In this manner, programmer 104 may receive updated stimulation parameter values (e.g., a different electrode combination to use for stimulation and/or recording) and transmit the updated stimulation parameters back to IMD 106. IMD 106 may then generate or receive updated stimulation parameters that define electrical stimulation and control stimulation circuitry of IMD 106 to deliver the electrical stimulation according to the updated stimulation parameters. In some examples, IMD 106 or programmer 104 may check whether pre-programmed groups or other parameter sets remain safe or effective with the new estimated location of the signal source. In some examples, IMD 106 and/or programmer 104 can confirm available parameter ranges are safe or appropriate with the new estimated location or alert a user when the moved lead is no longer compatible with the new estimated location with respect to the lead. IMD 106 and/or programmer 104 may inform the user directly or via a cloud-connected platform, for example. Alternatively, IMD 106 and/or programmer 104 may adjust available parameter value ranges in response to the changed electrode locations (e.g., due to the rotation and/or shift).

The architecture of system 100 illustrated in FIG. 1 is shown as an example. The techniques as set forth in this disclosure may be implemented in the example system 100 of FIG. 1, as well as other types of systems not described specifically herein. Nothing in this disclosure should be construed so as to limit the techniques of this disclosure to the example architecture illustrated by FIG. 1.

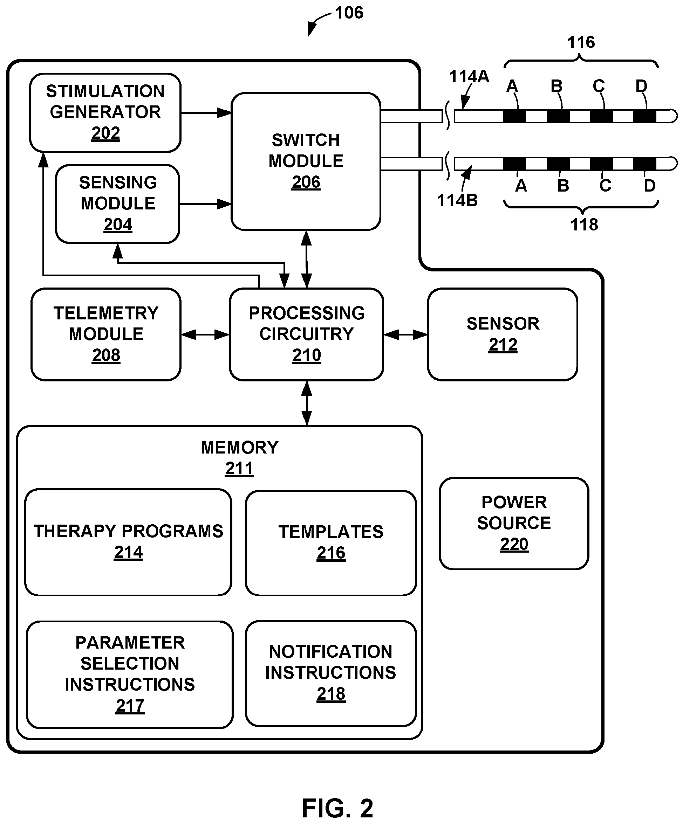

FIG. 2 is a block diagram of the example IMD 106 of FIG. 1 for delivering DBS therapy. In the example shown in FIG. 2, IMD 106 includes processing circuitry 210, memory 211, stimulation generator 202, sensing module 204, switch module 206, telemetry module 208, sensor 212, and power source 220. Each of these modules may be or include electrical circuitry configured to perform the functions attributed to each respective module. For example, processing circuitry 210 may include processing circuitry, switch module 206 may include switch circuitry, sensing module 204 may include sensing circuitry, and telemetry module 208 may include telemetry circuitry. Switch module 204 may not be necessary for multiple current source and sink configurations. Memory 211 may include any volatile or non-volatile media, such as a random-access memory (RAM), read only memory (ROM), non-volatile RAM (NVRAM), electrically erasable programmable ROM (EEPROM), flash memory, and the like. Memory 211 may store computer-readable instructions that, when executed by processing circuitry 210, cause IMD 106 to perform various functions. Memory 211 may be a storage device or other non-transitory medium.

In the example shown in FIG. 2, memory 211 stores therapy programs 214 that include respective stimulation parameter sets that define therapy. Each stored therapy program 214 defines a particular set of electrical stimulation parameters (e.g., a therapy parameter set), such as a stimulation electrode combination, electrode polarity, current or voltage amplitude, pulse width, and pulse rate. In some examples, individual therapy programs may be stored as a therapy group, which defines a set of therapy programs with which stimulation may be generated. The stimulation signals defined by the therapy programs of the therapy group may be delivered together on an overlapping or non-overlapping (e.g., time-interleaved) basis.

Memory 211 may also include templates 216 that define a relationship between one or more locations of a signal source with respect to one or more leads 114 and one or more features of sensed signals from one or more electrode combinations having electrodes carried by the one or more leads 114. In some examples, processing circuitry 210 may generate or otherwise obtain templates 216 from prior data. The prior data may be generated from modeling of sensed signals, experimental tank data, or information obtained from other patients. Memory 211 may also include parameter selection instructions 217 and notification instructions 218. Parameter selection instructions 217 may include instructions that control processing circuitry 210 selecting different stimulation parameter values such as electrode combinations, amplitudes, pulse frequencies, or other parameter values for compensating for lead movement. Parameter selection instructions 217 may include instructions for processing circuitry 210 to select parameter values based on the estimated location of a signal source determined by comparing sensed signals to one or more of templates 216. Notification instructions 218 may define instructions that control processing circuitry 210 actions such as transmitting an alert or other notification to an external device, such as programmer 104, that indicates a new location of a signal source has been detected (e.g., such as the lead has moved or condition has changed) or new parameter values have been determined. In some examples, notification instructions 218 may also define additional information that processing circuitry 210 transmits with the alert, such as an indication of which direction the lead moved, proposed electrode combinations closest to the target tissue after lead movement, or any other information that may assist the user in selecting new stimulation parameters.

In some examples, the sense and stimulation electrode combinations may include the same subset of electrodes 116, 118, a housing of IMD 106 functioning as an electrode, or may include different subsets or combinations of such electrodes. Thus, memory 211 can store a plurality of sense electrode combinations and, for each sense electrode combination, store information identifying the stimulation electrode combination that is associated with the respective sense electrode combination. The associations between sense and stimulation electrode combinations can be determined, e.g., by a clinician or automatically by processing circuitry 210. In some examples, corresponding sense and stimulation electrode combinations may comprise some or all of the same electrodes. In other examples, however, some or all of the electrodes in corresponding sense and stimulation electrode combinations may be different. For example, a stimulation electrode combination may include more electrodes than the corresponding sense electrode combination in order to increase the efficacy of the stimulation therapy. In some examples, as discussed above, stimulation may be delivered via a stimulation electrode combination to a tissue site that is different than the tissue site closest to the corresponding sense electrode combination but is within the same region, e.g., the thalamus, of brain 120 in order to mitigate any irregular oscillations or other irregular brain activity within the tissue site associated with the sense electrode combination. In other examples, the electrodes that deliver stimulation may be carried by a lead implanted in a different region of the brain than a different lead that carries the sensing electrodes.

Stimulation generator 202, under the control of processing circuitry 210, generates stimulation signals for delivery to patient 112 via selected combinations of electrodes 116, 118. An example range of electrical stimulation parameters believed to be effective in DBS to manage a movement disorder of patient include:

   1. Pulse Rate, i.e., Frequency: between approximately 0.1 Hertz and approximately 500 Hertz, such as between approximately 0.1 to 10 Hertz, approximately 40 to 185 Hertz, or such as approximately 140 Hertz.
   2. In the case of a voltage controlled system, Voltage Amplitude: between approximately 0.1 volts and approximately 50 volts, such as between approximately 2 volts and approximately 3 volts.
   3. In the alternative case of a current controlled system, Current Amplitude: between approximately 0.2 milliamps to approximately 100 milliamps, such as between approximately 1.3 milliamps and approximately 2.0 milliamps.
   4. Pulse Width: between approximately 10 microseconds and approximately 5000 microseconds, such as between approximately 100 microseconds and approximately 1000 microseconds, or between approximately 180 microseconds and approximately 450 microseconds.

Accordingly, in some examples, stimulation generator 202 generates electrical stimulation signals in accordance with the electrical stimulation parameters noted above. Other ranges of therapy parameter values may also be useful, and may depend on the target stimulation site within patient 112. While stimulation pulses are described, stimulation signals may be of any form, such as continuous-time signals (e.g., sine waves) or the like. Stimulation signals configured to elicit ECAPs or other evoked physiological signals may be similar or different from the above parameter value ranges.

Processing circuitry 210 may include fixed function processing circuitry and/or programmable processing circuitry, and may comprise, for example, any one or more of a microprocessor, a controller, a digital signal processor (DSP), an application specific integrated circuit (ASIC), a field-programmable gate array (FPGA), discrete logic circuitry, or any other processing circuitry configured to provide the functions attributed to processing circuitry 210 herein may be embodied as firmware, hardware, software or any combination thereof. Processing circuitry 210 may control stimulation generator 202 according to therapy programs 214 stored in memory 211 to apply particular stimulation parameter values specified by one or more of programs, such as voltage amplitude or current amplitude, pulse width, or pulse rate.

In the example shown in FIG. 2, the set of electrodes 116 includes electrodes 116A, 116B, 116C, and 116D, and the set of electrodes 118 includes electrodes 118A, 118B, 118C, and 118D. Processing circuitry 210 also controls switch module 206 to apply the stimulation signals generated by stimulation generator 202 to selected combinations of electrodes 116, 118. In particular, switch module 204 may couple stimulation signals to selected conductors within leads 114, which, in turn, deliver the stimulation signals across selected electrodes 116, 118. Switch module 206 may be a switch array, switch matrix, multiplexer, or any other type of switching module configured to selectively couple stimulation energy to selected electrodes 116, 118 and to selectively sense neurological brain signals with selected electrodes 116, 118. Hence, stimulation generator 202 is coupled to electrodes 116, 118 via switch module 206 and conductors within leads 114. In some examples, however, IMD 106 does not include switch module 206.

Stimulation generator 202 may be a single channel or multi-channel stimulation generator. In particular, stimulation generator 202 may be capable of delivering a single stimulation pulse, multiple stimulation pulses, or a continuous signal at a given time via a single electrode combination or multiple stimulation pulses at a given time via multiple electrode combinations. In some examples, however, stimulation generator 202 and switch module 206 may be configured to deliver multiple channels on a time-interleaved basis. For example, switch module 206 may serve to time divide the output of stimulation generator 202 across different electrode combinations at different times to deliver multiple programs or channels of stimulation energy to patient 112. Alternatively, stimulation generator 202 may comprise multiple voltage or current sources and sinks that are coupled to respective electrodes to drive the electrodes as cathodes or anodes. In this example, IMD 106 may not require the functionality of switch module 206 for time-interleaved multiplexing of stimulation via different electrodes.

Electrodes 116, 118 on respective leads 114 may be constructed of a variety of different designs. For example, one or both of leads 114 may include two or more electrodes at each longitudinal location along the length of the lead, such as multiple electrodes at different perimeter locations around the perimeter of the lead at each of the locations A, B, C, and D. On one example, the electrodes may be electrically coupled to switch module 206 via respective wires that are straight or coiled within the housing the lead and run to a connector at the proximal end of the lead. In another example, each of the electrodes of the lead may be electrodes deposited on a thin film. The thin film may include an electrically conductive trace for each electrode that runs the length of the thin film to a proximal end connector. The thin film may then be wrapped (e.g., a helical wrap) around an internal member to form the lead 114. These and other constructions may be used to create a lead with a complex electrode geometry.

Although sensing module 204 is incorporated into a common housing with stimulation generator 202 and processing circuitry 210 in FIG. 2, in other examples, sensing module 204 may be in a separate housing from IMD 106 and may communicate with processing circuitry 210 via wired or wireless communication techniques. Example neurological brain signals include, but are not limited to, a signal generated from local field potentials (LFPs) within one or more regions of brain 28. EEG and ECoG signals are examples of other types of electrical signals that may be measured within brain 120. Instead of, or in addition to, LFPs, IMD 106 may be configured to detect patterns of single-unit activity and/or multi-unit activity. IMD 106 may sample this activity at rates above 1,000 Hz, and in some examples within a frequency range of 6,000 Hz to 40,000 Hz. IMD 106 may identify the wave-shape of single units and/or an envelope of unit modulation that may be features used to differentiate or rank electrodes. In some examples, this technique may include phase-amplitude coupling to the envelope or to specific frequency bands in the LFPs sensed from the same or different electrodes.

Sensor 212 may include one or more sensing elements that sense values of a respective patient parameter. For example, sensor 212 may include one or more accelerometers, optical sensors, chemical sensors, temperature sensors, pressure sensors, or any other types of sensors. Sensor 212 may output patient parameter values that may be used as feedback to control delivery of therapy. IMD 106 may include additional sensors within the housing of IMD 106 and/or coupled via one of leads 114 or other leads. In addition, IMD 106 may receive sensor signals wirelessly from remote sensors via telemetry module 208, for example. In some examples, one or more of these remote sensors may be external to patient (e.g., carried on the external surface of the skin, attached to clothing, or otherwise positioned external to the patient). For example, IMD 106 may determine from these one or more additional sensors the brain state of the patient and sense signals for determining electrode movement during a brain state of lower fluctuation or lower noise to improve signal detection. In other examples, IMD 106 may employ an inertial sensor to determine when the patient is at rest (e.g., lying down and/or sleeping) and sense signals for determining lead movement during a time of rest to reduce noise or other motion artifacts in the sensed signals. In some examples, IMD 106 may sense signals for determining lead movement in response to receiving an indication that the patient received a dose of medication or the patient has entered a physician appointment.

Telemetry module 208 supports wireless communication between IMD 106 and an external programmer 104 or another computing device under the control of processing circuitry 210. Processing circuitry 210 of IMD 106 may receive, as updates to programs, values for various stimulation parameters such as magnitude and electrode combination, from programmer 104 via telemetry module 208. The updates to the therapy programs may be stored within therapy programs 214 portion of memory 211. In addition, processing circuitry 210 may control telemetry module 208 to transmit alerts or other information to programmer 104 that indicate a lead moved with respect to tissue. Telemetry module 208 in IMD 106, as well as telemetry modules in other devices and systems described herein, such as programmer 104, may accomplish communication by radiofrequency (RF) communication techniques. In addition, telemetry module 208 may communicate with external medical device programmer 104 via proximal inductive interaction of IMD 106 with programmer 104. Accordingly, telemetry module 208 may send information to external programmer 104 on a continuous basis, at periodic intervals, or upon request from IMD 106 or programmer 104.

Power source 220 delivers operating power to various components of IMD 106. Power source 220 may include a small rechargeable or non-rechargeable battery and a power generation circuit to produce the operating power. Recharging may be accomplished through proximal inductive interaction between an external charger and an inductive charging coil within IMD 220. In some examples, power requirements may be small enough to allow IMD 220 to utilize patient motion and implement a kinetic energy-scavenging device to trickle charge a rechargeable battery. In other examples, traditional batteries may be used for a limited period of time.

According to the techniques of the disclosure, processing circuitry 210 of IMD 106 delivers, electrodes 116, 118 interposed along leads 114 (and optionally switch module 206), electrical stimulation therapy to patient 112. The DBS therapy is defined by one or more therapy programs 214 having one or more parameters stored within memory 211. For example, the one or more parameters include a current amplitude (for a current-controlled system) or a voltage amplitude (for a voltage-controlled system), a pulse rate or frequency, and a pulse width, or quantity of pulses per cycle. In examples where the electrical stimulation is delivered according to a "burst" of pulses, or a series of electrical pulses defined by an "on-time" and an "off-time," the one or more parameters may further define one or more of a number of pulses per burst, an on-time, and an off-time.

When a lead rotates or shifts longitudinally, a different electrode combination may be best positioned to stimulate the tissue generating the neurological signal indicative of patient symptoms or of patient side-effects (e.g., the target region that may be the signal source of sensed signals). Therefore, as described herein, processing circuitry 210 (or a different processor of IMD 106 or a different device such as programmer 104) may be configured to determine an estimated location of the signal source in order to facilitate the selection of appropriate stimulation parameter values that define electrical stimulation to reduce or eliminate the patient symptoms and/or reduce or prevent patient side effects. Processing circuitry 210 may be configured to receive information representing a plurality of signals sensed from the tissue of the patient via a plurality of electrode combinations (e.g., electrode combinations that include two or more of electrodes 116 and/or 118).

Generally, the plurality of electrode combinations comprises different electrode combinations comprising electrodes disposed at different positions of the lead implanted in the patient. Processing circuitry 210 may determine one or more features from the information representing the plurality of signals and then compare the one or more features to a plurality of templates. Each template of the plurality of templates may represent respective locations of a signal source within the tissue. Processing circuitry 210 may then determine, based on the comparison of the one or more features to the plurality of templates, an estimated location of the signal source with respect to the lead.

In some examples, the plurality of electrode combinations include at least one electrode combination comprising electrodes disposed at different positions around a perimeter of the lead. In some examples, at least one electrode combination includes electrodes disposed at different positions along a longitudinal axis of the lead implanted in the patient. In this manner, the plurality of electrode combinations may include electrode combinations where each electrode combination has electrodes at different positions around the perimeter of the lead, electrode combinations where each electrode combination has electrode at different positions along the longitudinal axis of the lead, or electrode combinations that include both electrode combinations with electrodes at different positions around the perimeter of the lead and different positions along the longitudinal axis of the lead.

The one or more features determined from the sensed electrical signals may include or represent different characteristics of the sensed electrical signals. Example features of the sensed electrical signals may include voltage, impedance, spectral power, one or more frequencies, one or more frequency bands, or any other characteristics of the sensed signals. In this manner, the one or more features may be features in the time domain, frequency domain, or any other signal domain relevant to identify the sensed signals. For example, processing circuitry 210 may be configured to determine the one or more features by at least determining a sensed voltage for each electrode combination of the plurality of electrode combinations. In some examples, processing circuitry 210 may be configured to determine the one or more features by at least determining at least one of a power, a frequency band, a time domain feature, and/or a frequency domain feature of each signal of the plurality of signals from respective electrode combinations of the plurality of electrode combinations.

Processing circuitry 210 may store these one or more features for each electrode combination for use in comparing to the templates of estimated locations of the signal source. In one example, processing circuitry 210 may be configured to compare the sensed voltage for each electrode combination to template voltages of the plurality of templates. The different voltages from the different electrode combinations may be indicative of a certain location of the signal source with respect to the lead. For example, processing circuitry 210 may determine the estimated location by at least selecting the estimated location from one template of the plurality of templates that comprises template voltages closer to the sensed voltages than any other templates of the plurality of templates. In some examples, a look up table includes the plurality of templates, and each template includes template voltages for at least some of the plurality of electrode combinations corresponding to a respective location of a plurality of locations. In some examples, each template may include different types of features from the electrode combinations for each possible location identified by the templates. For example, each template may specify a relationship between different features and a corresponding location of the signal source with respect to the lead. A template may specify voltages for two or more electrode combinations, differential voltages between different electrode combinations, a spectral power of electrode combinations, or any other features. These features may be absolute in amplitude or normalized across all electrode combinations to be relatable to signals sources of varying amplitudes. In some examples, processing circuitry 210 may include cost functions or comparison functions that identify the estimated location based on the known feature variables.

In other examples, processing circuitry 210 may employ one or more machine learning techniques to determine the estimated location of a signal source with respect to a lead. For example, a machine learning algorithm may be trained using training data generated from computational models of electrical signals, experimental tank data, or signals obtained from other patients and known locations of signal sources. Once trained, processing circuitry 210 may apply the trained machine learning algorithm to the sensed signals from the plurality of electrode combinations to output an estimated location. Example machine learning algorithms may include classification methods, regression methods, unsupervised methods (e.g., cluster observations for association with a location or desired outcome), or any other type of machine learning algorithms.

Processing circuitry 210 may be configured to control a user interface to display the estimated location of the signal source with respect to a representation of anatomy and a representation of the lead. For example, processing circuitry 210 may transmit the estimated location and/or other data to programmer 104 for display to the user via the user interface. In some examples, processing circuitry 210 may be configured to receive user selection of a value for one or more stimulation parameters that at least partially defines electrical stimulation deliverable to the patient based on the estimated location. For example, the user interface may present a visual indication of the estimated location of the signal source in conjunction with one or more fields configured to accept user input on respective stimulation parameter values.

Processing circuitry 210 or other devices may generate the templates 216 and/or any equations or functions that define the relationships between features of electrical signals sensed by electrode combinations and possible locations of the signal source. For example, processing circuitry 210 may generate the plurality of templates from at least one of a mathematical model of locations distributed around the lead, experimental data, or data collected from prior patients different from the patient and store the plurality of templates in a memory. In some examples, the signals sensed by the electrode combinations are intrinsic physiological signals. In other examples, the sensed signals may be evoked by delivered electrical stimulus or other types of stimulus. In this manner, processing circuitry 210 may evoke the sensed signals in order to activate the signal source and identify the location. In some examples, processing circuitry 210 may determine the features of the sensed electrical features based on the difference between signals sensed prior to the delivered stimulus and the signals sensed after delivery of the stimulus. In this manner, the intrinsic signals may be background signals that can highlight the changes in the tissue due to the stimulus. The templates may be similarly generated to reflect this differential signal approach in order to be used to identify signal sources of evoked signals sensed by the different electrode combinations.

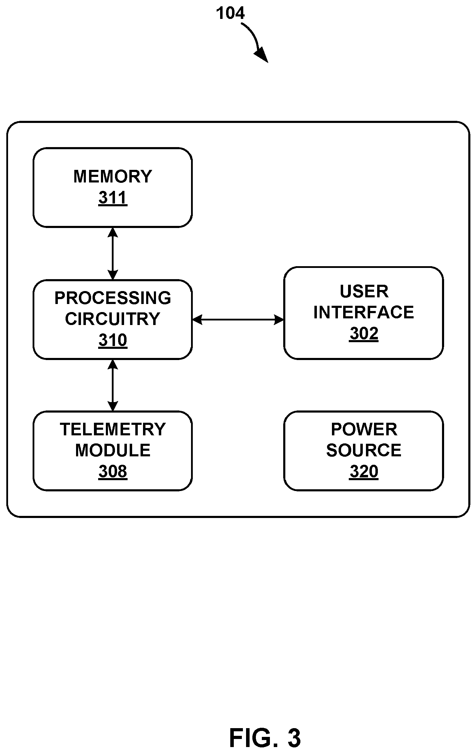

FIG. 3 is a block diagram of the external programmer 104 of FIG. 1 for controlling delivery of DBS therapy according to an example of the techniques of the disclosure. Although programmer 104 may generally be described as a hand-held device, programmer 104 may be a larger portable device or a more stationary device. In some examples, programmer 104 may be referred to as a tablet computing device. In addition, in other examples, programmer 104 may be included as part of a bed-side monitor, an external charging device or include the functionality of an external charging device. As illustrated in FIG. 3, programmer 104 may include a processing circuitry 310, memory 311, user interface 302, telemetry module 308, and power source 320. Memory 311 may store instructions that, when executed by processing circuitry 310, cause processing circuitry 310 and external programmer 104 to provide the functionality ascribed to external programmer 104 throughout this disclosure. Each of these components, or modules, may include electrical circuitry that is configured to perform some or all of the functionality described herein. For example, processing circuitry 310 may include processing circuitry configured to perform the processes discussed with respect to processing circuitry 310.

In general, programmer 104 comprises any suitable arrangement of hardware, alone or in combination with software and/or firmware, to perform the techniques attributed to programmer 104, and processing circuitry 310, user interface 302, and telemetry module 308 of programmer 104. In various examples, programmer 104 may include one or more processors, which may include fixed function processing circuitry and/or programmable processing circuitry, as formed by, for example, one or more microprocessors, DSPs, ASICs, FPGAs, or any other equivalent integrated or discrete logic circuitry, as well as any combinations of such components. Programmer 104 also, in various examples, may include a memory 311, such as RAM, ROM, PROM, EPROM, EEPROM, flash memory, a hard disk, a CD-ROM, comprising executable instructions for causing the one or more processors to perform the actions attributed to them. Moreover, although processing circuitry 310 and telemetry module 308 are described as separate modules, in some examples, processing circuitry 310 and telemetry module 308 may be functionally integrated with one another. In some examples, processing circuitry 310 and telemetry module 308 correspond to individual hardware units, such as ASICs, DSPs, FPGAs, or other hardware units.

Memory 311 (e.g., a storage device) may store instructions that, when executed by processing circuitry 310, cause processing circuitry 310 and programmer 104 to provide the functionality ascribed to programmer 104 throughout this disclosure. For example, memory 311 may include instructions that cause processing circuitry 310 to obtain a parameter set from memory, select a spatial electrode movement pattern, provide an interface that recommends or otherwise facilitates parameter value selection, or receive a user input and send a corresponding command to IMD 106, or instructions for any other functionality. In addition, memory 311 may include a plurality of programs, where each program includes a parameter set that defines stimulation therapy.

User interface 302 may include a button or keypad, lights, a speaker for voice commands, a display, such as a liquid crystal (LCD), light-emitting diode (LED), or organic light-emitting diode (OLED). In some examples the display may be a touch screen. User interface 302 may be configured to display any information related to the delivery of stimulation therapy, identified patient behaviors, estimated locations of signal sources, suggested stimulation parameter values, detected lead movement, sensed patient parameter values, patient behavior criteria, or any other such information. User interface 302 may also receive user input via user interface 302. The input may be, for example, in the form of pressing a button on a keypad or selecting an icon from a touch screen.

Telemetry module 308 may support wireless communication between IMD 106 and programmer 104 under the control of processing circuitry 310. Telemetry module 308 may also be configured to communicate with another computing device via wireless communication techniques, or direct communication through a wired connection. In some examples, telemetry module 308 provides wireless communication via an RF or proximal inductive medium. In some examples, telemetry module 308 includes an antenna, which may take on a variety of forms, such as an internal or external antenna. In some examples, IMD 106 and/or programmer 104 may communicate with remote servers via one or more cloud-services in order to deliver and/or receive information between a clinic and/or programmer.

Examples of local wireless communication techniques that may be employed to facilitate communication between programmer 104 and IMD 106 include RF communication according to the 802.11 or Bluetooth specification sets or other standard or proprietary telemetry protocols. In this manner, other external devices may be capable of communicating with programmer 104 without needing to establish a secure wireless connection. As described herein, telemetry module 308 may be configured to transmit a spatial electrode movement pattern or other stimulation parameter values to IMD 106 for delivery of stimulation therapy.

According to the techniques of the disclosure, in some examples, processing circuitry 310 of external programmer 104 may determine estimated locations of a signal source based on sensed electrical signals and templates as described herein. Programmer 104 may also determine stimulation parameter values based on the estimated location of the signal source. In one example, processor 311 of external programmer 104, via telemetry module 308, issues commands to IMD 106 causing IMD 106 to deliver electrical stimulation therapy via electrodes 116, 118 via leads 114.

FIGS. 4A and 4B are conceptual diagrams of example leads 400 and 410, respectively, with respective electrodes carried by the lead. As shown in FIGS. 4A and 4B, leads 400 and 410 are embodiments of leads 114 shown in FIG. 1. As shown in FIG. 4A, lead 400 includes four electrode levels 404 (includes levels 404A-404D) mounted at various lengths of lead housing 402. Lead 400 is inserted into through cranium 122 to a target position within brain 18.

Lead 400 is implanted within brain 120 at a location determined by the clinician to be near an anatomical region to be stimulated. Electrode levels 404A, 404B, 404C, and 404D are equally spaced along the axial length of lead housing 30 at different axial positions. Each electrode level 404 may have one, two, three, or more electrodes located at different angular positions around the circumference (e.g., around the perimeter) of lead housing 402. As shown in FIG. 4A, electrode level 404A and 404D include a single respective ring electrode, and electrode levels 404B and 404C each include three electrodes at different circumferential positions. This electrode pattern may be referred to as a 1-3-3-1 lead in reference to the number of electrodes from the proximal end to the distal end of lead 400. Electrodes of one circumferential location may be lined up on an axis parallel to the longitudinal axis of lead 400. Alternatively, electrodes of different electrode levels may be staggered around the circumference of lead housing 402. In addition, lead 400 or 410 may include asymmetrical electrode locations around the circumference, or perimeter, of each lead or electrodes of the same level that have different sizes. These electrodes may include semi-circular electrodes that may or may not be circumferentially aligned between electrode levels.

Lead housing 402 may include a radiopaque stripe or other one or more radiopaque marker (not shown) along the outside of the lead housing. The radiopaque stripe corresponds to a certain circumferential location that allows lead 400 to the imaged when implanted in patient 112. Using the images of patient 112, the clinician can use the radiopaque stripe as a marker for the exact orientation of lead 400 within the brain of patient 112. Orientation of lead 400 may be needed to easily program the stimulation parameters by generating the correct electrode configuration to match the stimulation field defined by the clinician. In other embodiments, a marking mechanism other than a radiopaque stripe may be used to identify the orientation of lead 400. These marking mechanisms may include something similar to a tab, detent, or other structure on the outside of lead housing 402. In some embodiments, the clinician may note the position of markings along a lead wire during implantation to determine the orientation of lead 400 within patient 112. In some examples, programmer 104 may update the orientation of lead 400 in visualizations based on the movement of lead 400 from sensed signals.

FIG. 4B illustrates lead 410 that includes multiple electrodes at different respective circumferential positions at each of levels 414A-414D. Similar to lead 400, lead 410 is inserted through a burr hole in cranium 122 to a target location within brain 120. Lead 410 includes lead housing 412. Four electrode levels 414 (414A-414D) are located at the distal end of lead 410. Each electrode level 414 is evenly spaced from the adjacent electrode level and includes two or more electrodes. In one embodiment, each electrode level 414 includes three, four, or more electrodes distributed around the circumference of lead housing 412. Therefore, lead 410 includes 414 electrodes in a preferred embodiment. Each electrode may be substantially rectangular in shape. Alternatively, the individual electrodes may have alternative shapes, e.g., circular, oval, triangular, rounded rectangles, or the like.

In alternative embodiments, electrode levels 404 or 414 are not evenly spaced along the longitudinal axis of the respective leads 400 and 410. For example, electrode levels 404C and 404D may be spaced approximately 3 millimeters (mm) apart while electrodes 404A and 404B are 10 mm apart. Variable spaced electrode levels may be useful in reaching target anatomical regions deep within brain 120 while avoiding potentially undesirable anatomical regions. Further, the electrodes in adjacent levels need not be aligned in the direction as the longitudinal axis of the lead, and instead may be oriented diagonally with respect to the longitudinal axis.

Leads 400 and 410 are substantially rigid to prevent the implanted lead from varying from the expected lead shape. Leads 400 or 410 may be substantially cylindrical in shape. In other embodiments, leads 400 or 410 may be shaped differently than a cylinder. For example, the leads may include one or more curves to reach target anatomical regions of brain 18. In some embodiments, leads 400 or 410 may be similar to a flat paddle lead or a conformable lead shaped for patient 12. Also, in other embodiments, leads 400 and 410 may any of a variety of different polygonal cross sections (e.g., triangle, square, rectangle, octagonal, etc.) taken transverse to the longitudinal axis of the lead.

As shown in the example of lead 400, the plurality of electrodes of lead 400 includes a first set of three electrodes disposed at different respective positions around the longitudinal axis of the lead and at a first longitudinal position along the lead (e.g., electrode level 404B), a second set of three electrodes disposed at a second longitudinal position along the lead different than the first longitudinal position (e.g., electrode level 404C), and at least one ring electrode disposed at a third longitudinal position along the lead different than the first longitudinal position and the second longitudinal position (e.g., electrode level 404A and/or electrode level 404D). In some examples, electrode level 404D may be a bullet tip or cone shaped electrode that covers the distal end of lead 402.

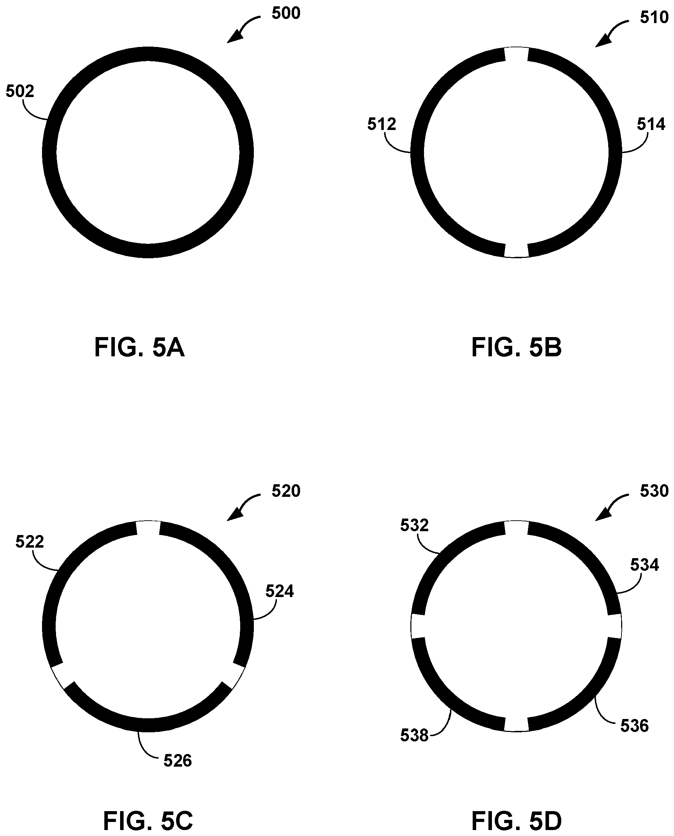

FIGS. 5A-5D are transverse cross-sections of example stimulation leads having one or more electrodes around the circumference of the lead. As shown in FIGS. 5A-5D, one electrode level, such as one of electrode levels 404 and 414 of leads 400 and 410, are illustrated to show electrode placement around the perimeter, or around the longitudinal axis, of the lead. FIG. 5A shows electrode level 500 that includes circumferential electrode 502. Circumferential electrode 502 encircles the entire circumference of electrode level 500 and may be referred to as a ring electrode in some examples. Circumferential electrode 502 may be utilized as a cathode or anode as configured by the user interface.

FIG. 5B shows electrode level 510 which includes two electrodes 512 and 514. Each electrode 512 and 514 wraps approximately 170 degrees around the circumference of electrode level 510. Spaces of approximately 10 degrees are located between electrodes 512 and 514 to prevent inadvertent coupling of electrical current between the electrodes. Smaller or larger spaces between electrodes (e.g., between 10 degrees and 30 degrees) may be provided in other examples. Each electrode 512 and 514 may be programmed to act as an anode or cathode.

FIG. 5C shows electrode level 520 which includes three equally sized electrodes 522, 524 and 526. Each electrode 522, 524 and 526 encompass approximately 110 degrees of the circumference of electrode level 520. Similar to electrode level 510, spaces of approximately 10 degrees separate electrodes 522, 524 and 526. Smaller or larger spaces between electrodes (e.g., between 10 degrees and 30 degrees) may be provided in other examples. Electrodes 522, 524 and 526 may be independently programmed as an anode or cathode for stimulation.

FIG. 5D shows electrode level 530 which includes four electrodes 532, 534, 536 and 538. Each electrode 532, 534, 536 and 538 covers approximately 80 degrees of the circumference with approximately 10 degrees of insulation space between adjacent electrodes. Smaller or larger spaces between electrodes (e.g., between 10 degrees and 30 degrees) may be provided in other examples. In other embodiments, up to ten or more electrodes may be included within an electrode level. In alternative embodiments, consecutive electrode levels of lead 114 may include a variety of electrode levels 500, 510, 520, and 530. For example, lead 114 (or any other lead described herein) may include electrode levels that alternate between electrode levels 510 and 530 depicted in FIGS. 5B and 5D. In this manner, various stimulation field shapes may be produced within brain 120 of patient 112. Further the above-described sizes of electrodes within an electrode level are merely examples, and the invention is not limited to the example electrode sizes.

Also, the insulation space, or non-electrode surface area, may be of any size. Generally, the insulation space is between approximately 1 degree and approximately 20 degrees. More specifically, the insulation space may be between approximately 5 and approximately 15 degrees. In other examples, insulation space may be between approximately 10 degrees and 30 degrees or larger. Smaller insulation spaces may allow a greater volume of tissue to be stimulated. In alternative embodiments, electrode size may be varied around the circumference of an electrode level. In addition, insulation spaces may vary in size as well. Such asymmetrical electrode levels may be used in leads implanted at tissues needing certain shaped stimulation fields.

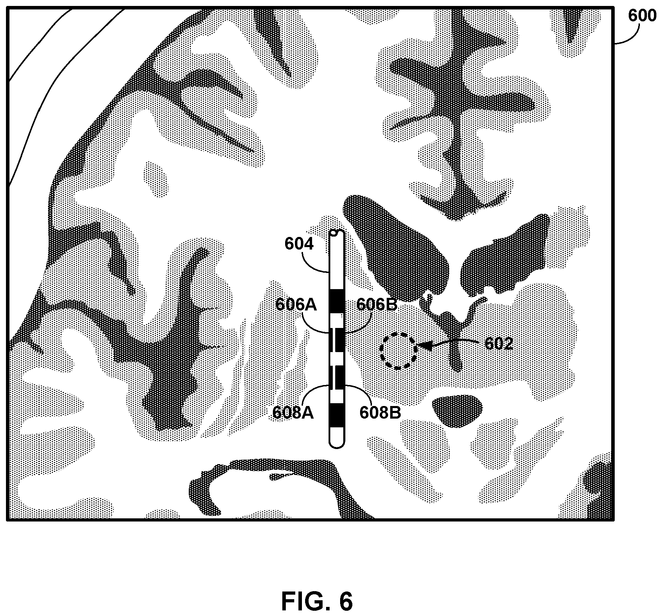

FIG. 6 is a coronal view of example tissue with a lead 604 placed with respect to a target location within tissue. As shown in FIG. 6, a representation of anatomical regions of brain 120 is displayed by coronal view 600. Coronal view 600 is a front-back vertical section of brain 120. Coronal view 600 may be an actual image of brain 120 produced with magnetic resonance imaging (MRI), computed tomography (CT), or another imaging modality. Coronal view 600 may be an illustration of the location of a lead with respect to a target tissue from which electrical signals originate (e.g., LFP signals). In some examples, coronal view 600 may be presented by programmer 104 or another device to indicate the relative position of lead 604 and the electrodes carried by the lead according to the sensed electrical signals. These images thus may be used to produce the anatomical regions needed to help the clinician program the stimulation parameters.

Coronal view 600 is a 2D coronal slice of brain 120. Differently shaded portions of coronal view 92 indicate varying densities of tissue within brain 120. Darker portions indicate less dense tissue. For example, the darkest portion of coronal view 600 is indicative of spaces within brain 120 that contain cerebral spinal fluid (CSF). White portions of brain 120 indicate dense tissue and more neurons. It should be noted that coronal view 600 is only an example, and actual images may include a wider range of shades and higher image resolution. Coronal view 600 provides a first perspective of the lead and the anatomical region in which the lead is implanted.

As shown in FIG. 6, lead 604 may be a lead icon that represents an actual lead implanted within patient 112. Lead 604 includes electrodes such as electrodes 606A and 606B located at the same longitudinal position and different circumferential positions around the perimeter of lead 604. Electrode 606C cannot be seen because it is located in the backside of lead 604. Similarly, lead 604 includes electrodes such as electrodes 608A and 608B located at the same longitudinal position and different circumferential positions around the perimeter of lead 604. Electrode 608C cannot be seen because it is located in the backside of lead 604. When electrical signals, such as LFP signals originate from target tissue 602 (e.g., a signal source), the largest amplitude and power of the signal will likely be sensed by the electrode or electrodes closest to target tissue 602, as this location may be associated with the largest therapeutic window. In this example, a sensing electrode combination of electrodes 606B and 608B may have the largest therapeutic window due to the location to target tissue 602 than any other electrode combinations on lead 604. In some examples, monopolar sensing may result in electrode 606B sensing the highest therapeutic window of electrical signals from target tissue 602. If lead 604 moves with respect to tissue, a different electrode, such as electrode 606A (for lead rotation) or electrode 608B (for longitudinal lead movement), may no longer have the largest therapeutic window.

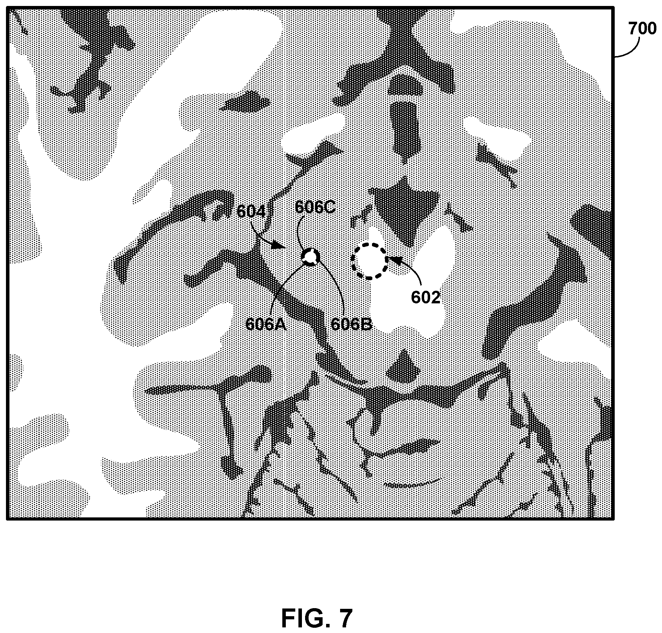

FIG. 7 is an axial view of example tissue with a lead 604 placed with respect to a target tissue 602 (e.g., a signal source). Axial view 700 is a different view of tissue than coronal view 600. Axial view 700 also shows the cross-sectional view of lead 604 and electrodes 606A, 606B, and 606C. As shown in axial view 700, electrode 606B is closest to target tissue 602 and may register the largest signal amplitudes when compared to the remaining electrodes of lead 604. If lead 604 were to rotate within tissue due to patient movement, lead pull, or some other force, a different electrode, such as electrode 606A, may be located closest to target tissue 602 and sense electrical signals with the largest amplitudes when compared to other electrodes. Lead 604 may rotate due to other factors as well, such as infection, subdural hematoma, stroke, seizure, post-implant tissue swelling, inflammation, relaxation of frictional sheer-forces between tissue and lead, changes in polymer properties, residual coiling forces in lead wiring, or any other causes. Although FIGS. 6 and 7 discuss electrical signals that may originate in tissue, the same spatial origin may be used when sensing electrical signals evoked from delivered stimulation or sensing delivered stimulation itself for determining lead movement.

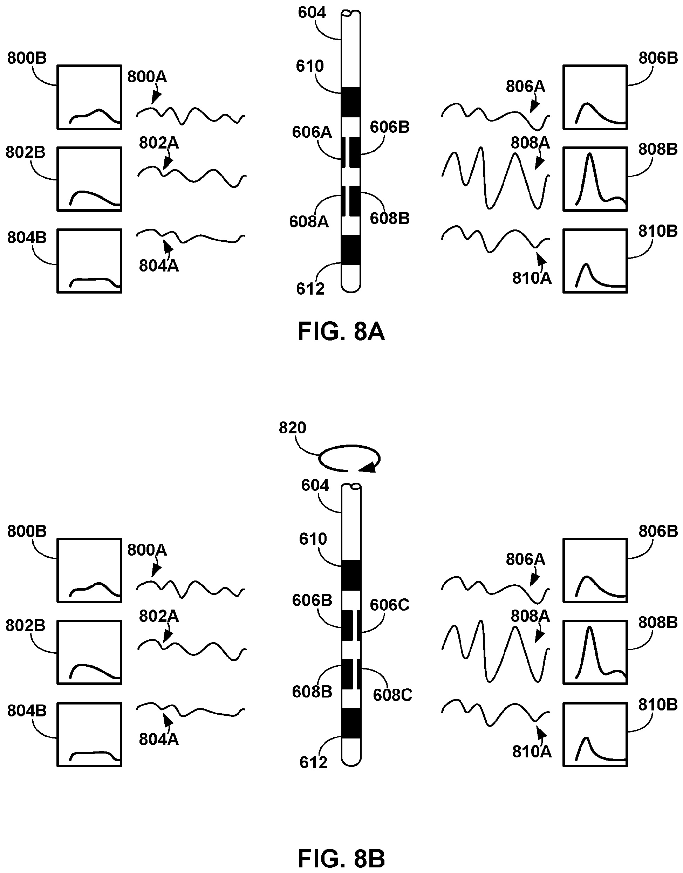

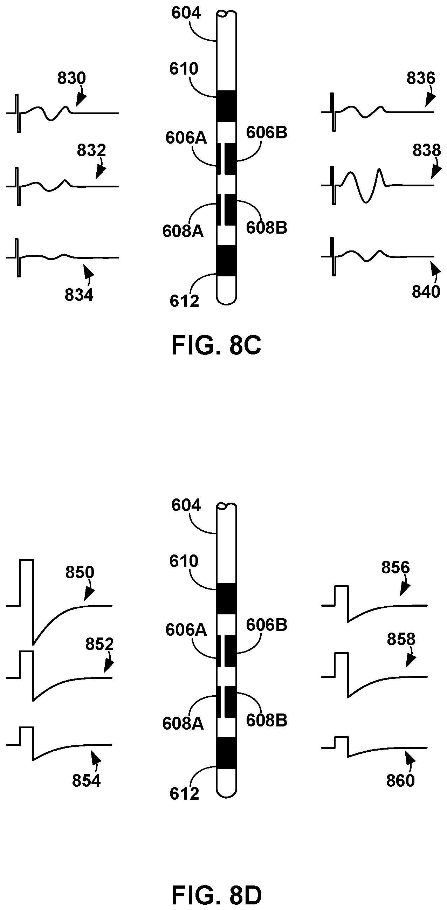

FIGS. 8A, 8B, 8C, and 8D are conceptual views of an example lead 604 with example initial information and signal information recorded from respective electrode combinations. As shown in FIG. 8A, initial information may represent electrical signals sensed at a first time when lead 604 is in a first position with respect to tissue. The initial information may include one or more features (e.g., characteristics) of the sensed electrical signals at the first time. The one or more features may include some aspect of the electrical signals that can be used to compare the electrode combinations to each initially and other over time to determine estimated locations of the signal source. For example, a feature may be an amplitude of the sensed signal (e.g., absolute amplitude, a normalized amplitude, a categorized amplitude (e.g., amplitude values fall within separate predetermined ranges), or a ranked amplitude). This amplitude may be the maximum amplitude over a period of time, for example. In other examples, the characteristic may be a differential signal between electrodes or a spatial derivative (e.g., first or second spatial derivative) in the axial and/or angular directions to estimate the proximity of each electrode to a signal source. For example, the second spatial derivative may provide information regarding how fast the signal amplitudes change to provide an indication of proximity of that electrode or electrode combination to the signal source. In other examples, a full two dimensional or three dimensional Laplacian can be employed for simultaneous recording across electrodes for the characteristic of each electrode and compared over time to estimate movements of lead 604 in all directions (e.g., rotational and shifting movements). These and other time domain features may be determined for each electrode or electrode combination used to sense the respective signals.

In some examples, the feature may be a relative phase between electrodes. The relative phase may differentiate between multiple tissue signal sources that may be out of phase with each other. IMD 106 may thus analyze the relative phase for each electrode or electrode combination and determine the orientation of the electrode(s) with respect to the signal source or sources. The relative phase may be employed by IMD 106 to improve the confidence in the lead orientation or lead movement determination in some examples. In other examples, the characteristic may be a spectral power or other feature in the frequency domain. The spectral power may be a power (e.g., absolute or normalized amplitude) for one or more frequency bands of the electrical signal. For example, IMD 106 may calculate the power of the beta frequency band for each sensed electrical signal, which may indicate the proximity of each electrode combination to a target neural location expected to generate signals in the beta frequency band. IMD 106 may select the frequency bad as generic for all patients or patient specific sensed signals. For example, the patient-specific frequency band may be selected to have a window centered around an identified peak in the spectrum (e.g., plus and minus 5 Hz from the identified peak). In other examples, IMD 106 may determine a rank for each electrode or electrode combination for any of the above-referenced parameters to determine if the rank of the electrodes changes between measurements or compared to determine which electrode combinations are closes to the signal source. In this manner, the initial information (and signal information) may include determined features representative of one or more aspects of the sensed electrical signals from each electrode combination. In addition, or alternatively, the initial information (and signal information) may include at least a portion of the sensed electrical signal waveform for comparison to a template, threshold, or some other function enabling comparison of the electrical signals sensed by different electrode combinations. Any of these characteristics may be used alone or in combination with other characteristics to identify an estimated location of a signal source and, in some examples, the electrode position with respect to a signal source and/or lead movement over time. In addition, any of these characteristics may be employed by IMD 106 to determine x, y, and z or r, theta, and z coordinates, depending on the desired coordinate system, of the signal source. IMD 106 may then determine the coordinates of the signal source (e.g., an estimated location) at multiple different times to identify any changes to the coordinates representative of lead movement (e.g., shift or rotation) and/or determine changes to one or more stimulation parameters needed due to different sensed signals.

In some examples, IMD 106 may perform corrections to sensed signals or include circuitry that balances impedance difference between electrodes of different sizes. This differences in impedances may alter the sensed signals and distort the determined distances to the signal source. IMD 106 may also compute corrections for different spacing between electrodes of different electrode combinations. For example, larger distances between electrodes similarly increases the amplitude of the sensed voltage. In order to compare signals from one electrode combination to another electrode combination with different spacing, IMD 106 may correct (or normalize) the sensed signal amplitude to compensate for these different spacings.

As shown in the example of FIG. 8A, waveform amplitudes 800A, 802A, 804A, 806A, 808A, and 810A (collectively "waveform amplitudes") are examples of initial information. Spectral powers 800B, 802B, 804B, 806B, 808B, and 810B (collectively "spectral powers") are additional, or alternative, examples of initial information. Each of the waveform amplitudes and spectral powers are determined from electrical signals sensed by a respective electrode combination. In the position of lead 604 as shown in the example of FIG. 8A, electrodes 606B and 608B are located on one side of lead 604 to detect signals such as waveform amplitudes 806A, 808A, and 810A and spectral powers 806B, 808B, and 810B. Conversely, electrodes 606A and 608A are located on a different side of lead 604 to detect signals such as waveform amplitudes 800A, 802A, and 804A and spectral powers 800B, 802B, and 804B. IMD 106 or another device may generate each of the spectral powers by analyzing the power of the frequencies present in the respective waveform amplitudes (e.g., spectral power 800B is generated by waveform amplitude 800A).

Each of the signals shown in FIG. 8A can be attributed to the signal sensed between an electrode combination of two electrodes. For example, IMD 106 may generate waveform amplitude 800A and/or spectral power 800B based on the electrical signal sensed between electrodes 610 and 606A. Similarly, IMD 106 may generate waveform amplitude 802A and/or spectral power 802B based on the electrical signal sensed between electrodes 606A and 608A and generate waveform amplitude 804A and/or spectral power 804B based on the electrical signal sensed between electrodes 608A and 612. Since electrodes 606A or 608A are part of the electrode combinations used to generate these signals, IMD 106 can determine that those signals originate from tissue in the direction of electrodes 606A or 608A. The same is true for other electrodes located at different positions around the perimeter of lead 604, such as electrodes 606B and 608B. Electrodes 606C and 608C are on the backside of lead 604 and cannot be viewed in the example of FIG. 8A. Therefore, IMD 106 may generate waveform amplitude 806A and/or spectral power 806B based on the electrical signal sensed between electrodes 610 and 606B. Similarly, IMD 106 may generate waveform amplitude 808A and/or spectral power 808B based on the electrical signal sensed between electrodes 606B and 608B and generate waveform amplitude 810A and/or spectral power 810B based on the electrical signal sensed between electrodes 608B and 612.

IMD 106 may monitor the signals sensed by the different electrode combinations over time to determine when the sensed signals, or characteristics of those signals, has changed indicating that lead 604 has rotated about the longitudinal axis and/or shifted along the longitudinal axis (e.g., the signal source has moved to a new location with respect to the lead). For example, as described herein, the features of the sensed electric signals from respective electrode combinations are compared to templates to identify the location of the signal source. In one example, the largest waveform amplitude and the largest spectral power as shown in FIG. 8A was waveform amplitude 808A and spectral power 808B from the signals sensed by the electrode combination of electrodes 606B and 608B. This larger amplitude may indicate that the tissue of interest is closest to electrodes 606B and 608B than any other electrodes of lead 604. The templates may convey this location information by incorporating the features from signals from multiple electrode combinations. In other examples, there may not be a tissue of interest. Instead, IMD 106 may use one or more of the characteristics of the signals sensed by the respective electrode combinations to establish a baseline pattern of signals that indicates an initial position in tissue of lead 604. IMD 106 may use this initial baseline pattern to detect changes in the signals sensed from one or more electrode combinations indicative of electrode movement with respect to tissue.

As shown in FIG. 8B, lead 604 has rotated in the direction of arrow 820 such that the electrode combinations of lead 604 sense different electrical signals in the new position of FIG. 8B than in the example of FIG. 8A. Put another way, the estimated location of the signal source will appear different with respect to the electrodes of lead 604. For example, IMD 106 may generate waveform amplitude 800A and/or spectral power 800B based on the electrical signal sensed between electrodes 610 and 606B. Similarly, IMD 106 may generate waveform amplitude 802A and/or spectral power 802B based on the electrical signal sensed between electrodes 606B and 608B and generate waveform amplitude 804A and/or spectral power 804B based on the electrical signal sensed between electrodes 608B and 612. Likewise, IMD 106 may generate waveform amplitude 806A and/or spectral power 806B based on the electrical signal sensed between electrodes 610 and 606B, generate waveform amplitude 808A and/or spectral power 808B based on the electrical signal sensed between electrodes 606C and 608C, and generate waveform amplitude 810A and/or spectral power 810B based on the electrical signal sensed between electrodes 608C and 612. IMD 106 may thus identify that the largest amplitude of waveform amplitude 808A or spectral power 808B is now associated with the electrode combination of electrodes 606C and 608C instead of by electrodes 606B and 608B by way of comparing features from the respective sensed signals from each electrode combination to one or more templates that output an estimated location of the signal source instead of, or in addition to, identifying the electrode combinations nearest to the signal source. By identifying the estimated location has changed, IMD 106 may thus identify that lead 604 has rotated in the direction of arrow 820. IMD 106 may thus adjust electrode combinations used to deliver therapy, adjust other therapy parameter values, initiate a reprogramming of therapy parameters, turn off therapy, or initiate an alert in response to determining that lead 604 has rotated with respect to tissue.

Although the same waveform amplitudes and spectral powers are shown in FIGS. 8A and 8B for illustrative purposes, the waveform amplitudes, spectral powers, or other features of sensed electrical signals may be different between different electrode combinations as a result of lead movement. For example, after lead 604 rotates, electrodes 606C and 608C may not sense the same electrical signals as sensed by electrodes 606B and 608B prior to the rotation. However, IMD 106 may trach the changes to the estimated location of the signal source that may result from the electrode combinations sensing signals having different features over time. In addition, the sensed signals shown in FIGS. 8A-8D may be used to generate stimulation parameter values and/or ranges of values for stimulation parameters that can be used to define stimulation therapy, such as which electrodes should be used for stimulation or appropriate current amplitudes, or even whether the lead has moved, or whether there is disease progression or changes.

Using the techniques described herein for identifying the estimated location of a signal source or changes to that location over time, IMD 106 may determine that lead 604 rotated in tissue or shifted up or down along the longitudinal axis of lead 604. IMD 106 may continually monitor electrode signals over time to identify additional movements of lead 604, such as rotations or shifts of lead 604 with respect to tissue. IMD 106 may determine lead movements based on electrical signals sensed by electrodes on any array of electrodes, which may encompass, one, two, three, or more separate leads. In this manner, IMD 106 may monitor any group of electrode combinations to determine when the electrodes have moved with respect to tissue.

The example features of the sensed electrical signals between electrodes of an electrode combination are the waveform amplitudes and spectral powers shown in FIGS. 8A and 8B. However, IMD 106 may determine estimated locations of a signal source based on different features of electrical signals. For example, IMD 106 may determine signal source locations based on changes to impedances sensed for one or more electrode combinations. IMD 106 may sense impedance between at least two electrodes of respective electrode combinations. Since the sensed impedance is affected by tissue between the electrodes, IMD 106 may determine signal source location based on changes to the sensed impedance of one or more electrode combinations.

Other types of sensed signals or features may be used by IMD 106 or another device to determine signal source location. As shown in FIG. 8C, IMD 106 may determine lead movement based on changes to sensed evoked responses (e.g., one or more characteristics of a physiologically generated electrical signal, such as an evoked compound action potential (ECAP)) sensed by one or more electrode combinations. IMD 106 may generate an electrical stimulus (e.g., a pulse or other waveform) via two or more electrodes on lead 604 or another lead. The electrical stimulus may be defined by stimulation parameters selected to elicit a nerve response that is detectable by electrodes on lead 604. The amplitude of the nerve response may be proportional to the distance from the stimulus electrodes and the nervous tissue and the distance from the nervous tissue and the sensing electrodes. Therefore, the amplitude of the sensed evoked signal may be indicative of how far the sensing electrodes are from the nervous tissue and whether or not the electrodes have moved with respect to the tissue. IMD 106 may sense evoked signals from all electrode combinations based on a single stimulus or sense evoked signals for each electrode combination from respective stimuli. IMD 106 may sense the evoked response on the same lead or a different lead than the lead which delivered the stimulation that elicited the evoked response. When stimulation is delivered by electrodes on one lead and evoked signals are sensed by electrodes on another lead, IMD 106 may determine from the sensed evoked signals whether the stimulation lead or the sensing lead has moved with respect to tissue.

For example, IMD 106 may generate waveform signal 830 based on the electrical signal sensed between electrodes 610 and 606A as a result of the delivered stimulus. Each sensed waveform signal, such as waveform signal 830, may include a first biphasic square wave pulse (e.g., an artifact representing the delivered stimulus) followed by the evoked signal from stimulated neural tissue. Similarly, IMD 106 may generate waveform signal 832 based on the electrical signal sensed between electrodes 606A and 608A and generate waveform signal 834 based on the electrical signal sensed between electrodes 608A and 612. Likewise, IMD 106 may generate waveform signal 836 based on the electrical signal sensed between electrodes 610 and 606B, generate waveform signal 838 based on the electrical signal sensed between electrodes 606B and 608B, and generate waveform signal 840 based on the electrical signal sensed between electrodes 608B and 612.

IMD 106 may analyze the amplitude, area under the curve, or other feature of at least a portion of the evoked waveform following the artifact. For example, the sensed signal may include multiple peaks of alternating polarities, and IMD 106 may analyze one or more of any of the peaks of the sensed signal. Similar to the waveform amplitude or spectral power discussed above, IMD 106 may monitor for changes to the evoked waveform feature, such as amplitude, over time. If the lead has moved, the sensed evoked response will change for one or more electrode combinations because the distance between the stimulus electrode(s) and the evoked tissue and/or the distance between the sensing electrodes and the evoked tissue will have changed. For example, evoked waveform 838 has the largest amplitude as sensed by electrodes 606B and 608B. If IMD 106 then determines that the electrode combination of electrodes 606A and 608A senses an evoked waveform that has the largest amplitude of all electrode combinations, then IMD 106 may determine that lead 604 has rotated such that electrodes 606A and 608A are now closer to the neuronal tissue than electrodes 606B and 608B. In some examples, the features determined for each electrode combination in the sensed evoked signal may be normalized by the features of the signals sensed prior to delivery of the stimulus. In this manner, only the change to tissue resulting in the evoked signal will result in the extracted features from the sensed evoked signals.

FIG. 8D illustrates another technique for determining signal source location that is similar to the evoked waveforms used in FIG. 8C. However, IMD 106 may determine signal source location based on changes to sensed electrical stimulus itself from one or more distant electrodes. IMD 106 may generate the electrical stimulus from an electrode on a housing of IMD 106, an electrode on a different lead, or control a different device (external or internal device) to deliver the electrical stimulus. IMD 106 may sense the electrical stimulus from one or more electrical combinations on lead 604, and this sensed signal may be referred to as an artifact or pulse because it is not physiological in origin. Nonetheless, electrodes closest to the origin of the electrical stimulus may sense the stimulus as a larger amplitude (e.g., a feature for comparison to one or more templates), indicative of the orientation of lead 604 with respect to the location of the electrical stimulus.

For example, IMD 106 may generate sensed signal 850 based on the electrical signal sensed between electrodes 610 and 606A indicative of the delivered stimulus. Each sensed signal, such as sensed signal 850, may include a first biphasic square wave pulse (e.g., an artifact representing the delivered stimulus). Similarly, IMD 106 may generate sensed signal 852 based on the electrical signal sensed between electrodes 606A and 608A and generate sensed signal 854 based on the electrical signal sensed between electrodes 608A and 612. Likewise, IMD 106 may generate sensed signal 856 based on the electrical signal sensed between electrodes 610 and 606B, generate sensed signal 858 based on the electrical signal sensed between electrodes 606B and 608B, and generate sensed signal 860 based on the electrical signal sensed between electrodes 608B and 612. In the example of FIG. 8D, IMD 106 may determine one or more features from the sensed signals for at least some of the electrode combinations and compare to one or templates indicative of the relationship between the features and possible locations of the signal source.

In this manner, if IMD 106 or another device delivers an electrical stimulus from an electrode not part of lead 604, for example, the electrode combinations of lead 604 will sense that delivered electrical stimulus with different respective amplitudes based on the orientation of the electrodes of each electrode combination with respect to the stimulus electrode. If the lead has moved, the sensed features will change for one or more electrode combinations because the distance between the stimulus electrode(s) and sensing electrodes of each electrode combination will have changed. IMD 106 may use any of these, or combinations of these, sensing techniques in order to determine movement of electrodes of a lead with respect to tissue.

In some examples, IMD 106, an external system (e.g., a lead trialing system), and/or a physician may implement the techniques described herein to intraoperatively rotate and/or shift the position of the lead to a target location with respect to surrounding anatomy. For example, the physician or surgical robot may rotate and/or shift the lead until an electrode, or electrode combination, is directly positioned to enable the electrodes to deliver stimulation to the target signal source. This positioning during implantation may enable the physician to improve the efficacy of stimulation, reduce side-effects, or increase the available therapeutic window (e.g., the greatest difference in amplitude between the minimum amplitude that provides therapy and the maximum amplitude that elicits side effects). To enable this intraoperative positioning technique, the lead may be connected to a trialing system similar to IMD 106, the IMD 106 itself, or an external recording system. If the lead is not directly connected to IMD 106, the physician may connect the lead to IMD 106 once positioned.

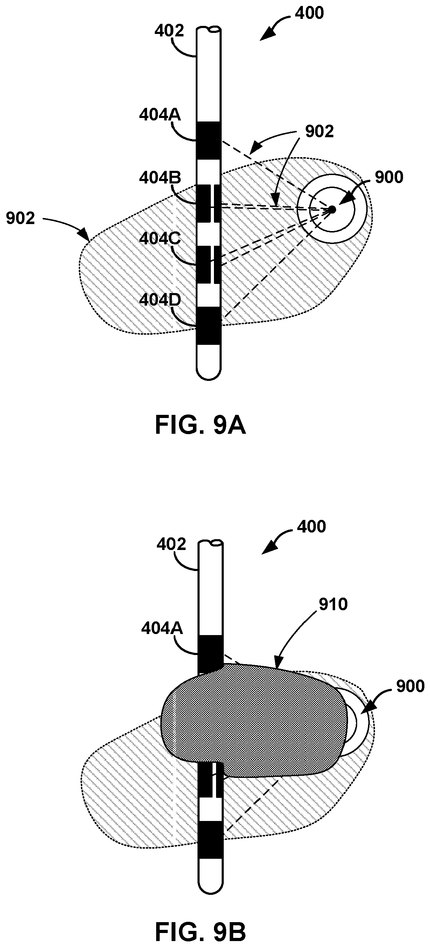

FIG. 9A is a conceptual illustration of an example signal source estimated location 900 with respect to electrodes 404 of example lead 402. As shown in the example of FIG. 9A, lead 402 is implanted partially within anatomical structure 902. Within anatomical structure 902, signal source 900 may be originating intrinsic signals. In order to identify the location of signal source 900, system 100 may sense electrical signals 902 from each of electrodes 404 or electrode combinations that include different sets of electrodes 404 and determine features from the sensed signals. System 100 may then compare or otherwise analyze the features with respect to one or more templates that define relationships between signal features and respective locations of signal sources with respect to lead 402.

The determined features from the sense signals from respective different electrode combinations may be described as a vector in equation 1 in some examples.

$$\vec{F}_{lead}(V_{E0}, V_{E1}, \dots) \hspace{4cm} \text{(Equation 1)}$$

In some examples, the comparison of the features to the templates may take the form of a cost function to minimize the difference from the features to identify the estimated location. An example cost function is provided in Equation 2. In Equation 2, the known features are those features obtained for respective known locations using prior data, such as using modeled data, experimentation tank data, prior patients, or any other known information.

$$g_{cost}(\mathbb{x})=\text{Cost}\left(\vec{F}_{lead},\left\{\vec{F}_{known}\,\middle|\,\mathbb{x}\right\}\right) \qquad \text{(Equation 2)}$$

Using the cost function in Equation 2, system 100 may determine the estimated location using Equation 3.

$$\widehat{\widehat{\mathbb{x}}}=\text{argmin}(g_{cost}(\mathbb{x})) \qquad \text{(Equation 3)}$$

In other examples, the comparison of the features to the templates may take the form of a comparison function to find the best match in values from the features to identify the estimated location. An example comparison function is provided in Equation 4.

$$g_{match}(\mathbb{x})=\text{Comparison}\left(\vec{F}_{lead},\left\{\vec{F}_{known}\,\middle|\,\mathbb{x}\right\}\right) \qquad \text{(Equation 4)}$$

Using the comparison function in Equation 5, system 100 may determine the estimated location using Equation 3.

$$\widehat{\widehat{\mathbb{x}}}=\text{argmax}(g_{match}(\mathbb{x})) \qquad \text{(Equation 5)}$$

These equations describe how the known features in the form of the templates can be used to determine the estimated location of a signal source using the features determined from the sensed signals from the patient. In some examples, system 100 may perform the matrix calculations to perform these comparisons. In other examples, system 100 may utilize a look up table to simplify the calculations during use for the patient.

Table 1 is an illustration of example templates of features for respective locations of a signal source in the form of a look up table. If using a comparison function, equation 5 may represent the different features that would correspond to respective locations.

**Table 1.**

| Location | E0-E1 | E 1-E2 | $V_i\text{-}V_j$ | ... |
|---|---|---|---|---|
| $r_1,\Theta_1, z_1$ | | | | |
| $r_2,\Theta_2, z_2$ | | | | |
| : | | | | |
| $\widehat{\widehat{\mathbb{x}}}=r_k,$ $\Theta_k, z_k$ | $\widehat{\widehat{\mathbb{x}}}=\text{argmax}(g(\mathbb{x}))$ | | | |
| : | | | | |
| $r_N,\Theta_N, z_N$ | | | | |

As shown in Table 1, each location may correspond to a set of different features obtained from the signals for different electrode combinations. The coordinates of each location can refer to the location with respect to the coordinate system of lead 400. The term "r" refers to the radius or distance away from the lead, the term "Θ" refers to the circumferential position around the perimeter of the lead, and the term "z" refers to the axial location along the longitudinal axis of the lead. The features E0-E1 and E1-E2 may represent the voltages sensed between a respective pair of electrodes, such as electrodes 0 and 1 and electrodes 1 and 2. The feature $V_i\text{-}V_j$ may correspond to the voltage difference between the voltages determined for two different electrodes. Additional or alternative features may be used in the templates in different examples. These vales in Table 1 may be normalized to compensate for different magnitudes of signal sources and instead provide an indication of how the different features change due to the geometric difference between the different electrodes and the location of a signal source. Using the look up table of Table 1, for example, system 100 may determine that signal source 900 has a location that corresponds the coordinates such as $r_k,\Theta_k, z_k$.

FIG. 9B is a conceptual illustrations of an example electrical field 910 that can be generated based on an estimated location of signal source 900. As shown in the example of FIG. 9B, system 100 or a clinician may determine a set of stimulation parameter values that generates electrical field 910 that reaches signal source 900, which may be the target tissue. In some examples, electrical field 910 may affect all or only a portion of the volume of signal source 900. System 100 or the clinician may weigh the benefits of reaching the entire volume of signal source 900 against possible side effects that can occur when the electrical field is increased to cover the entire volume of signal source 900. Instead of electrical field 910, system, 100 may refer to volume of activation, current density, or any other representation of the output of current from a set of electrodes delivering electrical stimulation.

System 100 may also display images similar to that shown in FIGS. 9A and/or 9B. In this manner, a display may present this information to visually represent the anatomy and stimulation field with respect to the location of the signal source 900. Although electrical field 910 is shown as a three-dimensional volume with respect to anatomical region 902 and signal source 900, electrical field 910 may be shown in one or more two-dimensional cross-sections or representations in other examples.

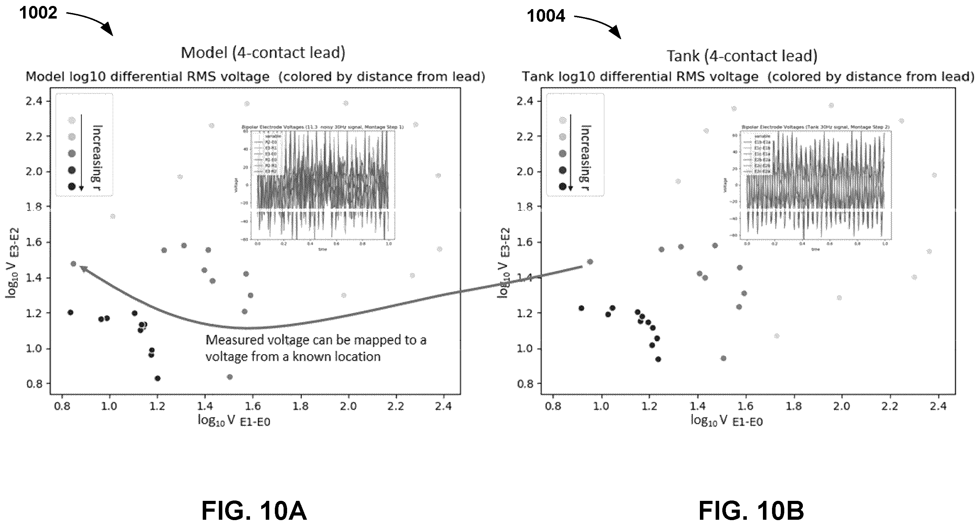

FIGS. 10A and 10B are example graphs 1002 ad 1004 illustrating example sensed voltages that vary with distance from electrode combinations. As shown in the examples of FIGS. 10A and 10B, sensed voltages for different electrode combinations were determined and plotted for different locations of a signal source. Graph 1002 illustrates a computational model of the sensed voltages for a four electrode lead. As shown in graph 1002, signal source locations further from the electrodes have lower voltages for the sensed signals. Graph 1004 is similar to graph 1002 but illustrates measured voltages from an electrode within an experimental tank from respective signal source locations. Graph 1002 and/or graph 1004 may be used to generate one or more templates of features corresponding to respective signal source locations with respect to the electrodes of the lead.

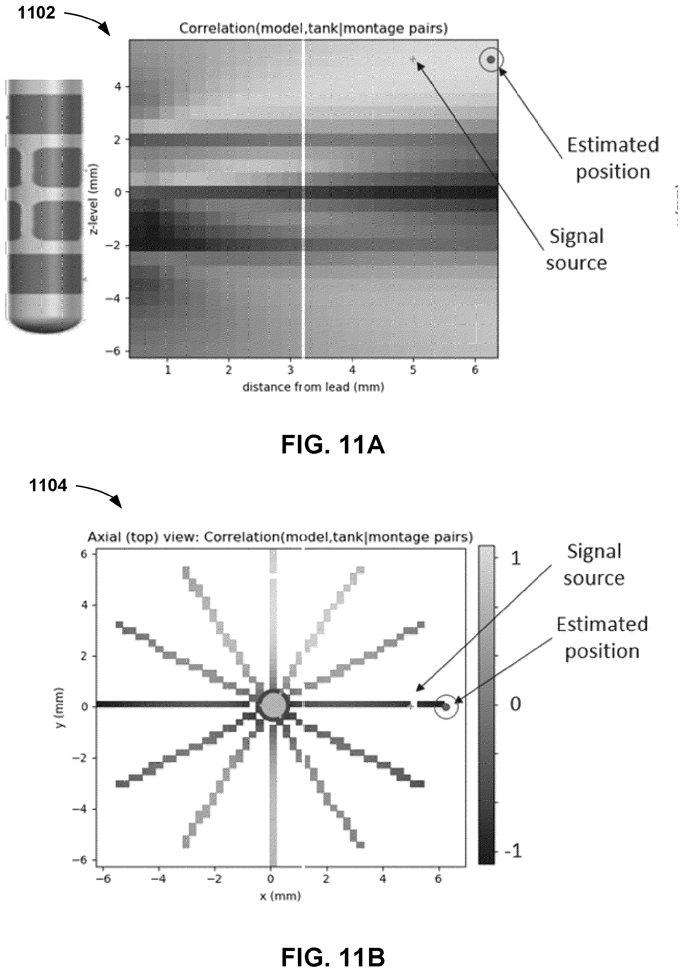

FIGS. 11A and 11B are example graph 1102 and 1004 illustrating estimated locations for a signal source compared with actual locations of the signal source. As shown in FIG. 11A, graph 1102 illustrates a correlation of signal features with respect to the distance away from the lead on the x-axis and the axial location of the lead on the y-axis. Graph 1102 shows a two-dimensional field extending away from the lead and indicates that the estimated location of the signal source to be about 6 mm away from the lead and 5 mm proximal from the center of the electrode array, which is close to the actual signal source location.

As shown in FIG. 11B, graph 1104 illustrates a correlation of signal features with respect to the distance away from the lead in one direction on the x-axis and the distance away from the lead in another direction on the y-axis. In this manner, the axial view of the lead in graph 1104 shows the lead in the middle. Graph 1102 indicates that the estimated location of the signal source to be about 6 mm away from the lead at a single circumferential location, which is close to the actual signal source location.

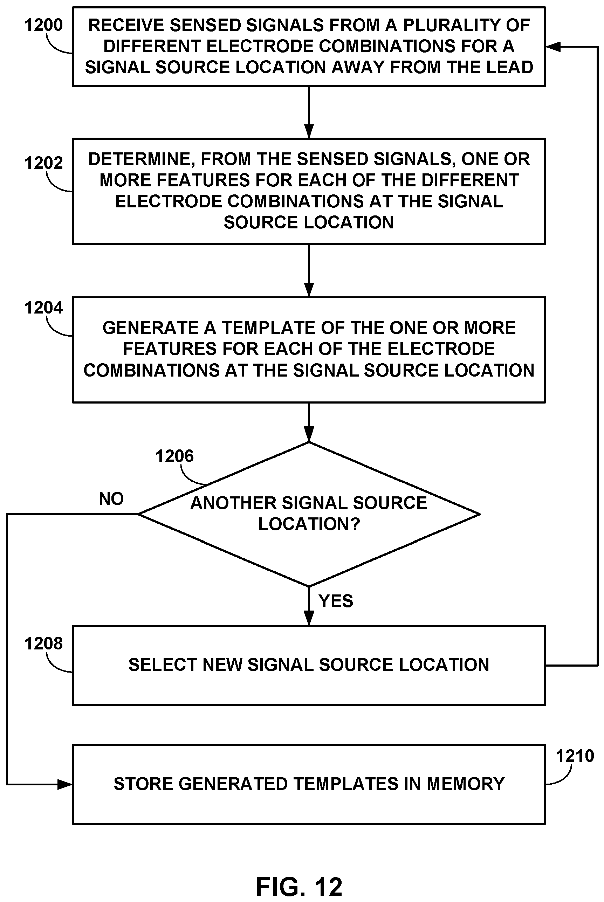

FIG. 12 is a flowchart illustrating an example technique for generating templates of one or more features from sensed signals for different electrode combinations and signal source locations with respect to a lead. The technique of FIG. 12 will be described with respect to processing circuitry 210 of IMD 106 in FIG. 2. However, other processors, devices, or combinations thereof, such as processing circuitry 310 of programmer 104 or some combination of devices or processors, may perform the techniques of FIG. 12 in other examples.

As shown in FIG. 12, processing circuitry 210 receives sensed signals from a plurality of different electrode combinations for a signal source location away from the lead (1200). Processing circuitry 210 the determines, from the sensed signals, one or more features for each of the different electrode combinations at the signal source location (1202). The sensed signals may be modeled from a computation model of anatomy and electrical field propagation in some examples, but the sensed signals may be physically sensed in an experimental tank in other examples. Alternatively, these sensed signals may be derived from signals obtained in other patients. For the signal source location, processing circuitry 210 generates a template of the one or more features for each of the electrode combinations at the signal source location (1204).

If there is another signal source location for which a template should be generated ("YES" branch of block 1206), processing circuitry 210 selects a new signal source location (1208) and then controls the sensing circuitry to receive sensed signals from the plurality of different electrode combinations for the new signal source location (1200). If there is no other signal source location for which a template should be generated ("NO" branch of block 1206), processing circuitry 210 then stores the generated templates in memory for use in later determination of signal source location based on sensed signals in a patient.

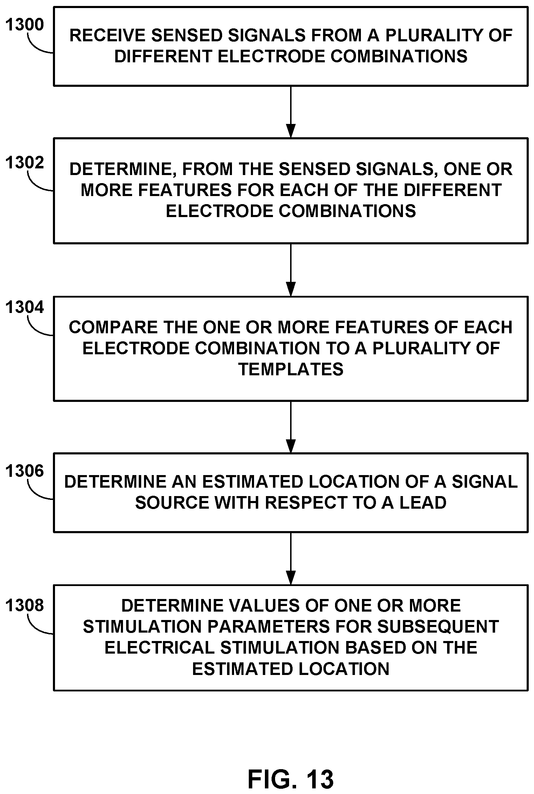

FIG. 13 is a flowchart illustrating an example technique for determining values of one or more stimulation parameters that define electrical stimulation deliverable based on the estimated location of a signal source with respect to a lead. The technique of FIG. 13 will be described with respect to processing circuitry 210 of IMD 106 in FIG. 2. However, other processors, devices, or combinations thereof, such as processing circuitry 310 of programmer 104 or some combination of devices or processors, may perform the techniques of FIG. 13 in other examples.

As shown in the example of FIG. 13, processing circuitry 210 receives sensed signals from a plurality of different electrode combinations of one or more leads (1300). Processing circuitry 210 the determines, from the sensed signals, one or more features for each of the different electrode combinations (1302). Example features may be in the time domain (e.g., voltage amplitudes) and/or in the frequency domain (e.g., spectral power in one or more frequency bands). Processing circuitry 210 then compares the one or more features of each electrode combination to a plurality of templates (1304). This comparison may be performed using a look up table or solving one or more equations, such as a cost function or comparison function. Based on the comparison of the one or more features to the templates, processing circuitry 210 then determines an estimated location of a signal source with respect to the one or more leads (1306). Processing circuitry 210 then determines values of one or more stimulation parameters that at least partially define subsequent electrical stimulation based on the estimated location of the signal source with respect to the lead (1308). Processing circuitry 210 can also control delivery of stimulation based on the stimulation parameter values.

In some examples, processing circuitry 210 may control a user interface to present a representation of the estimated location with respect to the lead to a user. This representation may be graphical or textual. The processing circuitry 210 may then receive user selection of one or more stimulation parameter values via a user interface after viewing the estimated location. Processing circuitry 210 may, in one example, present the determined values of the stimulation parameters via a user interface for confirmation from a user. In this way, the determined stimulation parameter values may be suggested values or recommended values that the user can approve or adjust as desired via the user interface. In addition or alternatively, processing circuitry 210 may perform the detection of the estimated location of the signal source repeatedly over time in order to identify any changes in the source location with respect to the lead. In response to detecting that the estimated location has changed, processing circuitry 210 may control a user interface to display an alert that the lead may have moved (e.g., axially or rotationally) with respect to tissue.

The techniques described in this disclosure may be implemented, at least in part, in hardware, software, firmware or any combination thereof. For example, various aspects of the described techniques may be implemented within one or more processors, such as fixed function processing circuitry and/or programmable processing circuitry, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit comprising hardware may also perform one or more of the techniques of this disclosure.

Such hardware, software, and firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

The techniques described in this disclosure may also be embodied or encoded in a computer-readable medium, such as a computer-readable storage medium, containing instructions. Instructions embedded or encoded in a computer-readable storage medium may cause a programmable processor, or other processor, to perform the method, e.g., when the instructions are executed. Computer readable storage media may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer readable media.

## Claims

1. A system comprising:
   processing circuitry (210) configured to:

   receive information representing a plurality of signals sensed from a tissue of a patient via a plurality of electrode combinations, wherein the plurality of electrode combinations comprises different electrode combinations comprising electrodes disposed at different positions of the lead implanted in the patient;
   determine one or more features from the information representing the plurality of signals;
   compare the one or more features to a plurality of templates, each template of the plurality of templates representing respective locations of a signal source within the tissue, wherein the signal source is an anatomical structure from which physiological signals originate or propagate and wherein each template includes a relationship between the one or more features and a corresponding location of the signal source with respect to the lead; and
   determine, based on the comparison of the one or more features to the plurality of templates, an estimated location of the signal source with respect to the lead.

2. The system of claim 1, wherein the plurality of electrode combinations comprises at least one electrode combination comprising electrodes disposed at different positions around a perimeter of the lead and at least one electrode combination comprising electrodes disposed at different positions along a longitudinal axis of the lead implanted in the patient.

3. The system of any of the preceding claims, wherein the processing circuitry (210) is configured to determine the one or more features by at least determining a sensed voltage for each electrode combination of the plurality of electrode combinations.

4. The system of claim 3, wherein the processing circuitry (210) is configured to:

compare the one or more features to the plurality of templates by at least comparing the sensed voltage for each electrode combination to template voltages of the plurality of templates; and
determine the estimated location by at least selecting the estimated location from one template of the plurality of templates that comprises template voltages closer to the sensed voltages than any other templates of the plurality of templates.

5. The system of claim 4, wherein a look up table comprises the plurality of templates, and wherein each template comprises template voltages for at least some of the plurality of electrode combinations corresponding to a respective location of a plurality of locations.

6. The system of any of the preceding claims, wherein the processing circuitry (210) is configured to determine the one or more features by at least determining at least one of a power, a frequency band, a time domain feature, or a frequency domain feature of each signal of the plurality of signals from respective electrode combinations of the plurality of electrode combinations.

7. The system of any of the preceding claims, wherein the processing circuitry (210) is configured to:

generate the plurality of templates from at least one of a mathematical model of locations distributed around the lead, experimental data, or data collected from prior patients different from the patient; and
store the plurality of templates in a memory.

8. The system of any of the preceding claims, wherein the processing circuitry (210) is configured to control a user interface to display the estimated location of the signal source with respect to a representation of anatomy and a representation of the lead.

9. The system of any of the preceding claims, wherein the processing circuitry (210) is configured to receive user selection of a value for one or more stimulation parameters that at least partially defines electrical stimulation deliverable to the patient based on the estimated location.

10. The system of any of the preceding claims, wherein the processing circuitry (210) is configured to determine, based on the estimated location of the signal source, one or more stimulation parameter values that at least partially define electrical stimulation therapy deliverable to the patient via the lead.

11. The system of any of the preceding claims, further comprising an implantable medical device (104) configured to deliver the electrical stimulation therapy to the patient via the lead and based on the estimated location.

12. A computer-readable storage medium, containing instructions, that when executed, cause processing circuitry to perform a method, the instructions comprising:

receiving, by processing circuitry, information representing a plurality of signals sensed from a tissue of a patient via a plurality of electrode combinations, wherein the plurality of electrode combinations comprises different electrode combinations comprising electrodes disposed at different positions of the lead implanted in the patient;
determining, by the processing, one or more features from the information representing the plurality of signals;
comparing, by the processing circuitry, the one or more features to a plurality of templates, each template of the plurality of templates representing respective locations of a signal source within the tissue, wherein the signal source is an anatomical structure from which physiological signals originate or propagate wherein each template includes a relationship between the one or more features and a corresponding location of the signal source with respect to the lead; and

determining, by the processing circuitry, based on the comparison of the one or more features to the plurality of templates, an estimated location of the signal source with respect to the lead.

13. The computer-readable storage medium of claim 12, wherein the plurality of electrode combinations comprises at least one electrode combination comprising electrodes disposed at different positions around a perimeter of the lead and at least one electrode combination comprising electrodes disposed at different positions along a longitudinal axis of the lead implanted in the patient.

14. The computer-readable storage medium of claim 12 or 13, wherein determining the one or more features comprises determining a sensed voltage for each electrode combination of the plurality of electrode combinations.

15. The computer-readable storage medium of claim 14, wherein:

comparing the one or more features to the plurality of templates comprises comparing the sensed voltage for each electrode combination to template voltages of the plurality of templates; and
determining the estimated location comprises selecting the estimated location from one template of the plurality of templates that comprises template voltages closer to the sensed voltages than any other templates of the plurality of templates.

**Patentansprüche**

1. System, umfassend:
eine Verarbeitungsschaltungsanordnung (210), die konfiguriert ist zum:

Empfangen von Informationen, die eine Vielzahl von Signalen darstellen, die von einem Gewebe eines Patienten über eine Vielzahl von Elektrodenkombinationen erfasst werden, wobei die Vielzahl von Elektrodenkombinationen unterschiedliche Elektrodenkombinationen umfasst, umfassend Elektroden, die an unterschiedlichen Positionen der Leitung eingerichtet sind, die in den Patienten implantiert ist;
Bestimmen eines oder mehrerer Merkmale aus den Informationen, die die Vielzahl von Signalen darstellen;
Vergleichen des einen oder der mehreren Merkmale mit einer Vielzahl von Vorlagen, wobei jede Vorlage der Vielzahl von Vorlagen jeweilige Standorte einer Signalquelle innerhalb des Gewebes darstellt, wobei die Signalquelle eine anatomische Struktur ist, von der physiologische Signale ausgehen oder sich ausbreiten, und wobei jede Vorlage eine Beziehung zwischen dem einen oder den mehreren Merkmale und einem entsprechenden Standort der Signalquelle in Bezug auf die Leitung einschließt; und
Bestimmen, basierend auf dem Vergleich des einen oder der mehreren Merkmale mit der Vielzahl von Vorlagen, eines geschätzten Standorts der Signalquelle in Bezug auf die Leitung.

2. System nach Anspruch 1, wobei die Vielzahl von Elektrodenkombinationen mindestens eine Elektrodenkombination, umfassend Elektroden, die an unterschiedlichen Standorten um einen Umfang der Leitung eingerichtet sind, und mindestens eine Elektrodenkombination umfasst, umfassend die Elektroden, die an unterschiedlichen Standorten entlang einer Längsachse der Leitung eingerichtet sind, die in den Patienten implantiert ist.

3. System nach einem der vorstehenden Ansprüche, wobei die Verarbeitungsschaltungsanordnung (210) konfiguriert ist, um das eine oder die mehreren Merkmale durch Bestimmen mindestens einer erfassten Spannung für jede Elektrodenkombination der Vielzahl von Elektrodenkombinationen zu bestimmen.

4. System nach Anspruch 3, wobei die Verarbeitungsschaltungsanordnung (210) konfiguriert ist zum:

Vergleichen des einen oder der mehreren Merkmale mit der Vielzahl von Vorlagen durch mindestens Vergleichen der erfassten Spannung für jede Elektrodenkombination mit Vorlagenspannungen der Vielzahl von Vorlagen; und
Bestimmen des geschätzten Standorts durch mindestens Auswählen des geschätzten Standorts aus einer Vorlage der Vielzahl von Vorlagen, die Vorlagenspannungen umfasst, die näher zu den erfassten Spannungen als alle anderen Vorlagen der Vielzahl von Vorlagen sind.

5. System nach Anspruch 4, wobei eine Nachschlagetabelle die Vielzahl von Vorlagen umfasst, und wobei jede Vorlage Vorlagenspannungen für mindestens einige der Vielzahl von Elektrodenkombinationen umfasst, die einem jeweiligen Standort einer Vielzahl von Standorten entsprechen.

6. System nach einem der vorstehenden Ansprüche, wobei die Verarbeitungsschaltungsanordnung (210) konfiguriert ist, um das eine oder mehreren Merkmale durch Bestimmen mindestens eines von einer Leistung, einem Frequenzband, einem Zeitbereichsmerkmal oder einem Frequenzbereichsmerkmal jedes Signals der Vielzahl von Signalen von jeweiligen Elektrodenkombinationen der Vielzahl von Elektrodenkombinationen zu bestimmen.

7. System nach einem der vorstehenden Ansprüche, wobei die Verarbeitungsschaltungsanordnung (210) konfiguriert ist zum:

Erzeugen der Vielzahl von Vorlagen aus mindestens einem von einem mathematischen Modell von Standorten, die um die Leitung verteilt sind, experimentellen Daten oder von Daten, die von früheren Patienten gesammelt wurden, die sich von dem Patienten unterscheiden; und
Speichern der Vielzahl von Vorlagen in einem Speicher.

8. System nach einem der vorstehenden Ansprüche, wobei die Verarbeitungsschaltungsanordnung (210) konfiguriert ist, um eine Benutzerschnittstelle zu steuern, um den geschätzten Standort der Signalquelle in Bezug auf eine Darstellung einer Anatomie und eine Darstellung der Leitung anzuzeigen.

9. System nach einem der vorstehenden Ansprüche, wobei die Verarbeitungsschaltungsanordnung (210) konfiguriert ist, um eine Benutzerauswahl eines Werts für einen oder mehrere Stimulationsparameter zu empfangen, der mindestens teilweise eine elektrische Stimulation definiert, die an den Patienten basierend auf dem geschätzten Standort abgebbar ist.

10. System nach einem der vorstehenden Ansprüche, wobei die Verarbeitungsschaltungsanordnung (210) konfiguriert ist, um basierend auf dem geschätzten Standort der Signalquelle einen oder mehrere Stimulationsparameterwerte zu bestimmen, die eine elektrische Stimulationstherapie mindestens teilweise definieren, die über die Leitung an den Patienten abgebbar ist.

11. System nach einem der vorstehenden Ansprüche, ferner umfassend eine implantierbare medizinische Vorrichtung (104), die konfiguriert ist, um die elektrische Stimulationstherapie über die Leitung und basierend auf dem geschätzten Standort an den Patienten abzugeben.

12. Computerlesbares Speichermedium, das Anweisungen enthält, die, wenn sie ausgeführt werden, eine Verarbeitungsschaltungsanordnung veranlassen, ein Verfahren durchzuführen, die Anweisungen umfassend:

Empfangen, durch eine Verarbeitungsschaltungsanordnung, von Informationen, die eine Vielzahl von Signalen darstellen, die von einem Gewebe eines Patienten über eine Vielzahl von Elektrodenkombinationen erfasst werden, wobei die Vielzahl von Elektrodenkombinationen unterschiedliche Elektrodenkombinationen umfasst, umfassend Elektroden, die an unterschiedlichen Positionen der Leitung eingerichtet sind, die in den Patienten implantiert ist;
Bestimmen, durch das Verarbeiten, eines oder mehrerer Merkmale aus den Informationen, die die Vielzahl von Signalen darstellen;
Vergleichen, durch die Verarbeitungsschaltungsanordnung, des einen oder der mehreren Merkmale mit einer Vielzahl von Vorlagen, wobei jede Vorlage der Vielzahl von Vorlagen jeweilige Standorte einer Signalquelle innerhalb des Gewebes darstellt, wobei die Signalquelle eine anatomische Struktur ist, von der physiologische Signale stammen oder sich ausbreiten, wobei jede Vorlage eine Beziehung zwischen dem einen oder den mehreren Merkmalen und einer entsprechenden Position der Signalquelle in Bezug auf die Leitung einschließt; und
Bestimmen, durch die Verarbeitungsschaltungsanordnung, basierend auf dem Vergleich des einen oder der mehreren Merkmale mit der Vielzahl von Vorlagen, eines geschätzten Standorts der Signalquelle in Bezug auf die Leitung.

13. Computerlesbares Speichermedium nach Anspruch 12, wobei die Vielzahl von Elektrodenkombinationen mindestens eine Elektrodenkombination, umfassend Elektroden, die an unterschiedlichen Positionen um einen Umfang der Leitung eingerichtet sind, und mindestens eine Elektrodenkombination umfasst, umfassend Elektroden, die an unterschiedlichen Positionen entlang einer Längsachse der Leitung eingerichtet sind, die in den Patienten implantiert ist.

**14.** Computerlesbares Speichermedium nach Anspruch 12 oder 13, wobei das Bestimmen des einen oder der mehreren Merkmale das Bestimmen einer erfassten Spannung für jede Elektrodenkombination der Vielzahl von Elektrodenkombinationen umfasst.

**15.** Computerlesbares Speichermedium nach Anspruch 14, wobei:

das Vergleichen des einen oder der mehreren Merkmale mit der Vielzahl von Vorlagen das Vergleichen der erfassten Spannung für jede Elektrodenkombination mit Vorlagenspannungen der Vielzahl von Vorlagen umfasst; und
das Bestimmen der geschätzten Position das Auswählen der geschätzten Position aus einer Vorlage der Vielzahl von Vorlagen umfasst. die Vorlagenspannungen umfasst, die näher zu den erfassten Spannungen als alle anderen Vorlagen der Vielzahl von Vorlagen sind.

**Revendications**

**1.** Système comprenant :
un circuit de traitement (210) configuré pour :

recevoir des informations représentant une pluralité de signaux détectés à partir d'un tissu d'un patient par l'intermédiaire d'une pluralité de combinaisons d'électrodes, dans lequel la pluralité de combinaisons d'électrodes comprend différentes combinaisons d'électrodes comprenant des électrodes disposées à différentes positions de la sonde implantée dans le patient ;
déterminer une ou plusieurs caractéristiques à partir des informations représentant la pluralité de signaux ;
comparer l'une ou les caractéristiques à une pluralité de modèles, chaque modèle de la pluralité de modèles représentant des emplacements respectifs d'une source de signal à l'intérieur du tissu, dans lequel la source de signal est une structure anatomique à partir de laquelle les signaux physiologiques proviennent ou se propagent et dans lequel chaque modèle comporte une relation entre l'une ou les caractéristiques et un emplacement correspondant de la source de signal par rapport à la sonde ; et
déterminer, en fonction de la comparaison de l'une ou des caractéristiques à la pluralité de modèles, un emplacement estimé de la source de signal par rapport à la sonde.

**2.** Système selon la revendication 1, dans lequel la pluralité de combinaisons d'électrodes comprend au moins une combinaison d'électrodes comprenant des électrodes disposées à différentes positions autour d'un périmètre de la sonde et au moins une combinaison d'électrodes comprenant des électrodes disposées à différentes positions le long d'un axe longitudinal de la sonde implantée dans le patient.

**3.** Système selon l'une quelconque des revendications précédentes, dans lequel le circuit de traitement (210) est configuré pour déterminer l'une ou les caractéristiques en déterminant au moins une tension détectée pour chaque combinaison d'électrodes de la pluralité de combinaisons d'électrodes.

**4.** Système selon les revendications 3, dans lequel le circuit de traitement (210) est configuré pour :

comparer l'une ou les caractéristiques à la pluralité de modèles en comparant au moins la tension détectée pour chaque combinaison d'électrodes à des tensions de modèle de la pluralité de modèles ; et
déterminer l'emplacement estimé en sélectionnant au moins l'emplacement estimé à partir d'un modèle de la pluralité de modèles qui comprend des tensions de modèle plus proches des tensions détectées que tout autre modèle de la pluralité de modèles.

**5.** Système selon la revendication 4, dans lequel une table de consultation comprend la pluralité de modèles, et dans lequel chaque modèle comprend des tensions de modèle pour au moins certaines de la pluralité de combinaisons d'électrodes correspondant à un emplacement respectif d'une pluralité d'emplacements.

**6.** Système selon l'une quelconque des revendications précédentes, dans lequel le circuit de traitement (210) est configuré pour déterminer l'une ou les caractéristiques en déterminant au moins l'une parmi une puissance, une bande de fréquences, une caractéristique de domaine temporel ou une caractéristique de domaine fréquentiel de chaque signal de la pluralité de signaux à partir de combinaisons d'électrodes respectives de la pluralité de combinaisons d'électrodes.

7. Système selon l'une quelconque des revendications précédentes, dans lequel le circuit de traitement (210) est configuré pour :

générer la pluralité de modèles à partir d'au moins l'un parmi un modèle mathématique d'emplacements répartis autour de la sonde, des données expérimentales, ou des données collectées auprès de patients antérieurs différents du patient ; et
stocker la pluralité de modèles dans une mémoire.

8. Système selon l'une quelconque des revendications précédentes, dans lequel le circuit de traitement (210) est configuré pour commander une interface utilisateur pour afficher l'emplacement estimé de la source de signal par rapport à une représentation de l'anatomie et à une représentation de la sonde.

9. Système selon l'une quelconque des revendications précédentes, dans lequel le circuit de traitement (210) est configuré pour recevoir une sélection utilisateur d'une valeur pour un ou plusieurs paramètres de stimulation qui définissent au moins partiellement la stimulation électrique administrable au patient en fonction de l'emplacement estimé.

10. Système selon l'une quelconque des revendications précédentes, dans lequel le circuit de traitement (210) est configuré pour déterminer, en fonction de l'emplacement estimé de la source de signal, une ou plusieurs valeurs de paramètres de stimulation qui définissent au moins partiellement la thérapie de stimulation électrique administrable au patient par l'intermédiaire de la sonde.

11. Système selon l'une quelconque des revendications précédentes, comprenant en outre un dispositif médical implantable (104) configuré pour administrer la thérapie de stimulation électrique au patient par l'intermédiaire de la sonde et en fonction de l'emplacement estimé.

12. Support de stockage lisible par ordinateur, contenant des instructions, qui lorsqu'elles sont exécutées, amènent le circuit de traitement à effectuer un procédé, les instructions comprenant :

la réception, par un système de circuit de traitement, des informations représentant une pluralité de signaux détectés à partir d'un tissu d'un patient par l'intermédiaire d'une pluralité de combinaisons d'électrodes, dans lequel la pluralité de combinaisons d'électrodes comprend différentes combinaisons d'électrodes comprenant des électrodes disposées à différentes positions de la sonde implantée dans le patient ;
la détermination, par le traitement, d'une ou plusieurs caractéristiques à partir des informations représentant la pluralité de signaux ;
la comparaison, par le circuit de traitement, de l'une ou des caractéristiques à une pluralité de modèles, chaque modèle de la pluralité de modèles représentant des emplacements respectifs d'une source de signal à l'intérieur du tissu, dans lequel la source de signal est une structure anatomique à partir de laquelle les signaux physiologiques proviennent ou se propagent, dans lequel chaque modèle comporte une relation entre l'une ou les caractéristiques et un emplacement correspondant de la source de signal par rapport à la sonde ; et
la détermination, par le circuit de traitement, en fonction de la comparaison de l'une ou des caractéristiques à la pluralité de modèles, d'un emplacement estimé de la source de signal par rapport à la sonde.

13. Support de stockage lisible par ordinateur selon la revendication 12, dans lequel la pluralité de combinaisons d'électrodes comprend au moins une combinaison d'électrodes comprenant des électrodes disposées à différentes positions autour d'un périmètre de la sonde et au moins une combinaison d'électrodes comprenant des électrodes disposées à différentes positions le long d'un axe longitudinal de la sonde implantée dans le patient.

14. Support de stockage lisible par ordinateur selon la revendication 12 ou 13, dans lequel la détermination de l'une ou des caractéristiques comprend la détermination d'une tension détectée pour chaque combinaison d'électrodes de la pluralité de combinaisons d'électrodes.

15. Support de stockage lisible par ordinateur selon la revendication 14, dans lequel :

la comparaison de l'une ou des caractéristiques à la pluralité de modèles comprend la comparaison de la tension détectée pour chaque combinaison d'électrodes à des tensions de modèle de la pluralité de modèles ; et
la détermination de l'emplacement estimé comprend la sélection de l'emplacement estimé à partir d'un modèle de la pluralité de modèles qui comprend des tensions de modèle plus proches des tensions détectées que tout autre modèle de la pluralité de modèles.

FIG. 1

FIG. 2

104

MEMORY
311

PROCESSING
CIRCUITRY
310

USER
INTERFACE
302

TELEMETRY
MODULE
308

POWER
SOURCE
320

FIG. 3

400

402

404A

404B

404C

404D

410

412

414A

414B

414C

414D

## FIG. 4A

## FIG. 4B

FIG. 5A

FIG. 5B

FIG. 5C

FIG. 5D

**FIG. 6**

**FIG. 7**

**FIG. 8A**

**FIG. 8B**

830

832

834

604

610

606A  606B

608A  608B

612

836

838

840

**FIG. 8C**

850

852

854

604

610

606A  606B

608A  608B

612

856

858

860

**FIG. 8D**

**FIG. 9A**

**FIG. 9B**

FIG. 10A

FIG. 10B

**FIG. 11A**

**FIG. 11B**

```
┌──────────────────────────────────────────────────┐
│  RECEIVE SENSED SIGNALS FROM A PLURALITY OF        │
1200 │  DIFFERENT ELECTRODE COMBINATIONS FOR A           │
│  SIGNAL SOURCE LOCATION AWAY FROM THE LEAD         │
└──────────────────────────────────────────────────┘
                        │
                        ▼
┌──────────────────────────────────────────────────┐
│  DETERMINE, FROM THE SENSED SIGNALS, ONE OR        │
1202 │  MORE FEATURES FOR EACH OF THE DIFFERENT          │
│  ELECTRODE COMBINATIONS AT THE SIGNAL             │
│  SOURCE LOCATION                                   │
└──────────────────────────────────────────────────┘
                        │
                        ▼
┌──────────────────────────────────────────────────┐
1204 │  GENERATE A TEMPLATE OF THE ONE OR MORE           │
│  FEATURES FOR EACH OF THE ELECTRODE              │
│  COMBINATIONS AT THE SIGNAL SOURCE LOCATION       │
└──────────────────────────────────────────────────┘
                        │
                        ▼
                1206
        NO    ╱ ANOTHER SIGNAL SOURCE ╲
        ◄─────   LOCATION?            
                ╲                     ╱
                        │
                       YES
                        ▼
┌──────────────────────────────────────────────────┐
1208 │          SELECT NEW SIGNAL SOURCE LOCATION        │
└──────────────────────────────────────────────────┘

┌──────────────────────────────────────────────────┐
│       STORE GENERATED TEMPLATES IN MEMORY    1210 │
└──────────────────────────────────────────────────┘
```

FIG. 12

| 1300 | RECEIVE SENSED SIGNALS FROM A PLURALITY OF DIFFERENT ELECTRODE COMBINATIONS |

| 1302 | DETERMINE, FROM THE SENSED SIGNALS, ONE OR MORE FEATURES FOR EACH OF THE DIFFERENT ELECTRODE COMBINATIONS |

| 1304 | COMPARE THE ONE OR MORE FEATURES OF EACH ELECTRODE COMBINATION TO A PLURALITY OF TEMPLATES |

| 1306 | DETERMINE AN ESTIMATED LOCATION OF A SIGNAL SOURCE WITH RESPECT TO A LEAD |

| 1308 | DETERMINE VALUES OF ONE OR MORE STIMULATION PARAMETERS FOR SUBSEQUENT ELECTRICAL STIMULATION BASED ON THE ESTIMATED LOCATION |

**FIG. 13**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018071530 A1